(19) Europäisches Patentamt
European Patent Office
Office européen des brevets

(11) Veröffentlichungsnummer: **0 254 859**
**B1**

(12) # EUROPÄISCHE PATENTSCHRIFT

(45) Veröffentlichungstag der Patentschrift: **01.08.90**

(21) Anmeldenummer: **87108747.4**

(22) Anmeldetag: **19.06.87**

(51) Int. Cl.⁵: **C07D 213/61**, C07D 261/08, C07D 275/02, C07D 213/38, C07D 213/64, C07D 231/12, A01N 43/00, A01N 43/40

(54) **Alkylendiamine.**

(30) Priorität: **01.07.86 JP 152763/86**

(43) Veröffentlichungstag der Anmeldung:
**03.02.88 Patentblatt 88/5**

(45) Bekanntmachung des Hinweises auf die Patenterteilung:
**01.08.90 Patentblatt 90/31**

(84) Benannte Vertragsstaaten:
**BE CH DE FR GB IT LI NL**

(56) Entgegenhaltungen:
**EP-A- 0 154 178**
**EP-A- 0 163 855**
**EP-A- 0 192 060**

**ADV. PESTIC. CHEM.**

(73) Patentinhaber: **NIHON TOKUSHU NOYAKU SEIZO K.K., Itohpia Nihonbashi Honcho Building 7-1, Nihonbashi Honcho 2-chome, Chuo-ku Tokyo 103(JP)**

(72) Erfinder: **Shiokawa, Kozo, 210-6, Shukugawara Tama-Ku, Kawasaki-shi Kanagawa-ken(JP)**
Erfinder: **Tsuboi, Shinichi, 3-26-1, Hirayama, Hino-shi Tokyo(JP)**
Erfinder: **Kagabu, Shinzo, 1768-532, Sagiyama, Gifu-shi Gifu-ken(JP)**
Erfinder: **Sasaki, Shoko, 1-7-3, Higashi-Hirayama, Hino-shi Tokyo(JP)**
Erfinder: **Moriya, Koichi, 5-7-11, Ueno, Taito-ku Tokyo(JP)**
Erfinder: **Hattori, Yumi, 598, Kobiki-cho, Hachioji-shi Tokyo(JP)**

(74) Vertreter: **Ernst, Hilmar, Dr. et al, Bayer AG Konzernverwaltung RP Patentabteilung, D-5090 Leverkusen 1, Bayerwerk(DE)**

**EP 0 254 859 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

## Beschreibung

Die vorliegende Erfindung betrifft neue Alkylendiamine, Verfahren zu ihrer Herstellung und ihre Verwendung als Insektizide.

Es wurden neue Alkylendiamine der Formel (I)

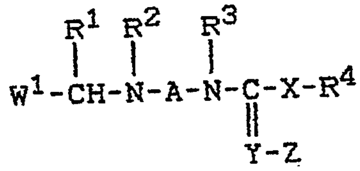 (I),

gefunden, in welcher
$R^1$, $R^2$ und $R^3$ Wasserstoff oder $C_{1-4}$-Alkyl bezeichnen,
$R^4$ Wasserstoff, $C_{1-4}$-Alkyl, $C_{6-10}$-Aryl, Benzyl, Phenethyl, $C_{1-4}$-Alkoxy, Dialkylamino mit insgesamt 2 bis 6-Kohlenstoff-Atomen, Alkoxyalkyl mit insgesamt 2 bis 6 Kohlenstoff-Atomen, Alkylthioalkyl mit insgesamt 2 bis 4 Kohlenstoff-Atomen oder eine Gruppe der Formel $-CH_2-W^2$ bezeichnet, in der $W^2$ die gleichen Bedeutungen hat, wie sie hiernach für $W^1$ angegeben sind,
X S oder

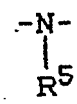

bezeichnet, worin $R^5$
Wasserstoff oder $C_{1-4}$-Alkyl bezeichnet, wobei in dem Fall, in dem X

dem Fall, in dem X 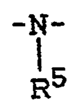 ist, die Gruppe 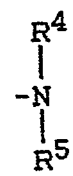 in

der Formel (I) die gleiche Bedeutung wie die Gruppe

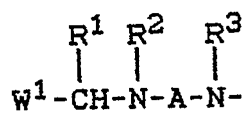

in der Formel (I) haben kann,
Y N oder

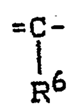

$C_{1-4}$-Alkyl, $C_{6-10}$-Aryl, $C_{1-4}$-Alkylcarbonyl, Alkyoxycarbonyl mit $C_{1-4}$-Alkoxy oder Cyano bezeichnet,
Z Cyano oder Nitro bezeichnet,
$W^1$ eine 5- oder 6-gliedrige heterocyclische Gruppe mit 1 bis 3 Hetero-Atomen ausgewählt aus O, S und N, von denen wenigstens eines N ist, bezeichnet, wobei die Gruppe $W^1$ gegebenenfalls durch wenigstens einen Substituenten ausgewählt aus Halogen, $C_{1-4}$-Alkyl, $C_{1-4}$-Alkoxy, $C_{1-4}$-Alkylthio, $C_{1-4}$-Halogenalkyl und $C_{1-4}$-Halogenalkoxy substituiert ist, und
A Ethylen, das gegebenenfalls durch Methyl substituiert sein kann, oder Trimethylen, das gegebenenfalls durch Methyl substituiert sein kann, bezeichnet.

Die Verbindungen der Formel (I) werden mit Hilfe eines Verfahrens erhalten, bei dem

a) in dem Fall, in dem in der Formel (I) X S ist und $R^4$ andere Bedeutungen als "Alkoxy" und "Dialkylamino" in den Definitionen hat, wobei in diesem Fall in der nachstehenden Formel (III) $R^4$ durch

das Symbol $R^7$ ersetzt ist, vorausgesetzt, daß die zwei Substituenten $R^7$ nicht gleichzeitig Wasserstoff sind, Verbindungen der Formel (II)

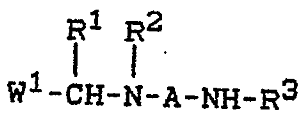 (II),

in der $R^1$, $R^2$, $R^3$, $W^1$ und A die angegebenen Bedeutungen haben, mit Verbindungen der Formel (III)

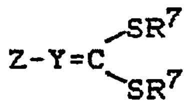 (III),

in der Y, Z und $R^7$ die angegebenen Bedeutungen haben, in Gegenwart inerter Lösungsmittel umgesetzt werden, oder

b) in dem Fall, in dem in der Formel (I)
X

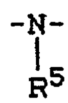

ist,
Verbindungen der Formel (IV)

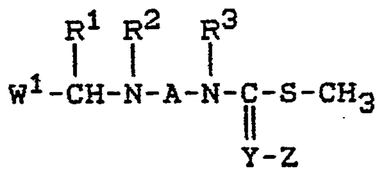 (IV),

in der $R^1$, $R^2$, $R^3$, Y, Z, $W^1$ und A die angegebenen Bedeutungen haben, mit Verbindungen der Formel (V)

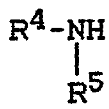 (V),

in der $R^4$ und $R^5$ die angegebenen Bedeutungen haben, in Gegenwart inerter Lösungsmittel umgesetzt werden, oder

c) in dem Fall, in dem in der Formel (I)

X 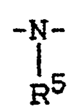

ist und Y N ist,
oben erwähnte Verbindungen der Formel (II) mit Verbindungen der Formel (IV)

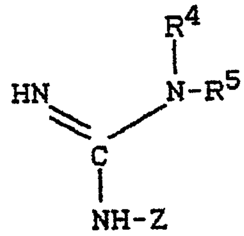

(VI),

in der $R^4$, $R^5$ und Z die angegebenen Bedeutungen haben, in Gegenwart inerter Lösungsmittel umgesetzt werden, oder

d) in dem Fall, in dem in der Formel (I)

Y 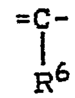

ist, X eine Einfachbindung ist und $R^4$ durch das oben bezeichnete Symbol $R^7$ ersetzt ist, oben erwähnte Verbindungen der Formel (II) mit Verbindungen der Formel (VII)

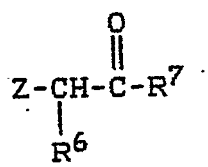

(VII)

in der $R^6$, $R^7$ und Z die angegebenen Bedeutungen haben, in Gegenwart inerter Lösungsmittel umgesetzt werden, oder

e) in dem Fall, in dem in der Formel (I) X S ist und $R^4$ andere Bedeutungen als "Wasserstoff", "Alkoxy" und "Dialkylamino" in den Definitionen hat, wobei in diesem Fall in der nachstehenden Formel (IX) $R^4$ durch das Symbol $R^8$ ersetzt ist, Verbindungen der Formel (VIII)

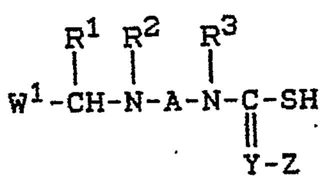

(VIII)

in der $R^1$, $R^2$, $R^3$ Y, Z und $W^1$ die angegebenen Bedeutungen haben, mit Verbindungen der Formel (IX)

Hal–$R^8$ (IX),

in der $R^8$ die angegebene Bedeutung hat und Hal ein Halogen bezeichnet, in Gegenwart inerter Lösungsmittel und gegebenenfalls in Gegenwart einer Base umgesetzt werden.

Die neuen Alkylendiamine zeigen potente insektizide Eigenschaften, insbesondere gegen saugende Insekten, wie sie typisch durch Insekten der Gattung Hemiptera repräsentiert werden, etwa durch Blattläuse, Laternenträger, Wanzen und Heuschrecken, die infolge einer Langzeit-Anwendung Resistenz gegen Organophosphat- und Carbamat-Insektizide erworben haben.

Aus folgenden Publikationen: EP-A 0 163 855, EP-A 0 192 060 und Adv. Pestic. Chem. 4th Int. Congr. Pest. Chem., Vol. 2, S. 206–217 (1979) sind ebenfalls Nitromethylen-Insektizide bekannt, die jedoch im Gegensatz zu den offenkettigen erfindungsgemäßen Verbindungen strukturell unterschiedliche ringgeschlossene Verbindungen betreffen.

Die Eignung der erfindungsgemäßen Alkylendiamine der allgemeinen Formel (I) als potente Insektizide war daher neu und überraschend.

Unter den neuen erfindungsgemäßen Alkylendiaminen der Formel (I) sind bevorzugte Verbindungen solche, in denen

$R^1$, $R^2$ und $R^3$ Wasserstoff, Methyl oder Ethyl bezeichnen,
$R^4$ ein Wasserstoff-Atom, Methyl, Ethyl, Phenyl, Benzyl, Methoxy, Dimethylamino, 1-Ethoxyethyl, 1-Ethylthioethyl oder 2-Chloro-5-pyridylmethyl bezeichnet,
X S oder

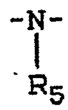

bezeichnet, worin $R^5$
Wasserstoff oder Methyl bezeichnet,
Y N oder

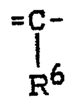

bezeichnet, worin $R^6$ Wasserstoff,
Methyl, Phenyl, Acetyl, Ethoxycarbonyl oder Cyano bezeichnet,
Z Cyano oder Nitro bezeichnet,
$W^1$ eine 5- oder 6-gliedrige heterocyclische Gruppe mit 1 bis 2 Hetero-Atomen ausgewählt aus O, S und N, von denen wenigstens eines N ist, bezeichnet, wobei die Gruppe $W^1$ gegebenenfalls durch wenigstens einen Substituenten ausgewählt aus Fluoro, Chloro, Bromo, Methyl, Methoxy, Methylthio, Trifluoromethyl und Trifluoromethoxy substituiert ist, und
A Ethylen oder Trimethylen bezeichnet.

Zu speziellen Beispielen für die Verbindungen der Formel (I) zählen unter anderem:
N-(2-Chloro-5-pyridylmethyl)-N-Methy-N′-(1-methylthio-2-nitrovinyl)ethylendiamin,
N-(2-Chloro-5-pyridylmethyl)-N-ethyl-N′-(1-methylthio-2-nitrovinyl)ethylendiamin,
N-(2-Chloro-5-pyridylmethyl)-N′-(1-mercapto-2-nitrovinyl)ethylerndiamin,
N-(2-Chloro-5-pyridylmethyl)-N′-(1-mercapto-2-nitrovinyl)trimethylendiamin,
N-(2-Chloro-5-pyridylmethyl)-N-methyl-N′-(1-ethylthio-2-nitrovinyl)ethylendiamin und
N-(2-Chloro-5-pyridylmethyl)-N-methyl-N′-(1-propylthio-2-nitrovinyl)ethylendiamin.

Wenn in dem Verfahren a) N-(2-Chloro-5-pyridylmethyl)-N-methylethylendiamin und 1-Nitro-2,2-bis(methylthio)-ethylen, beispielsweise, als Ausgangsstoffe eingesetzt werden, wird das Verfahren durch das folgende Reaktionsschema dargestellt:

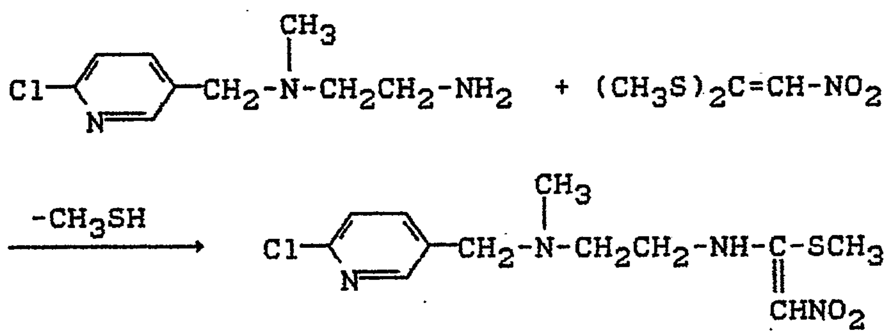


Wenn im Verfahren b) N-(2-Chloro-5-pyridylmethyl)-N-methyl-N′-(1-methylthio-2-nitrovinyl)ethylendiamin und O-Methylhydroxylamin, beispielsweise, als Ausgangsstoffe eingesetzt werden, wird das Verfahren durch das folgende Reaktionsschema dargestellt:

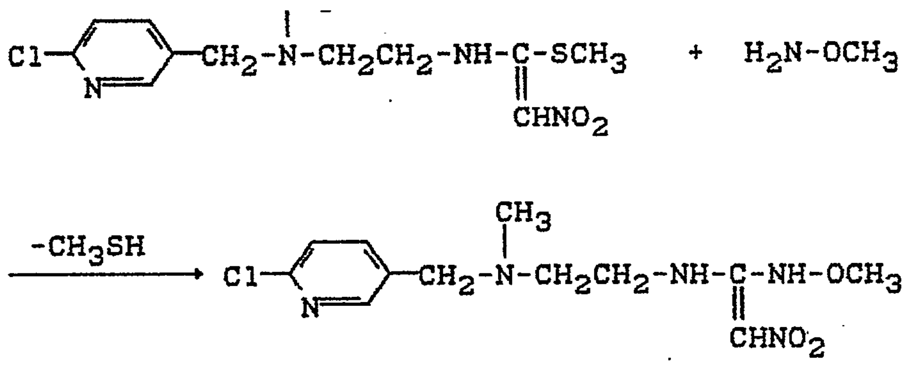


Wenn im Verfahren c) N-(2-Chloro-5-pyridylmethyl)-N-methylethylendiamin und N,N-dimethylnitroguanidin, beispielsweise, als Ausgangsstoffe eingesetzt werden, wird das Verfahren durch das folgende Reaktionsschema dargestellt:

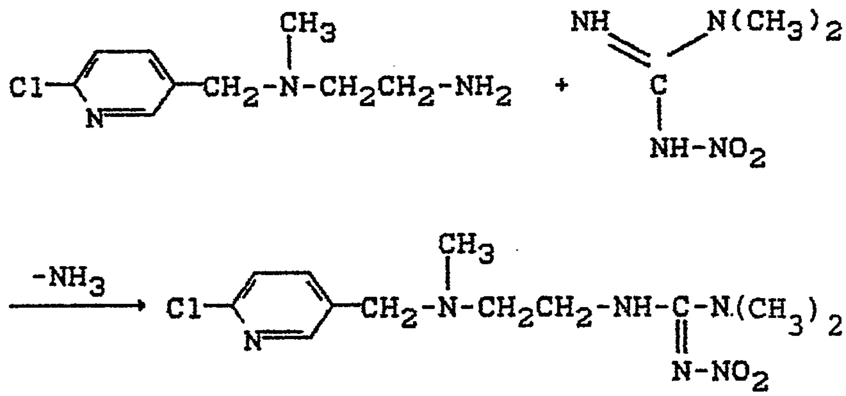


Wenn im Verfahren d) N-(2-Chloro-5-pyridylmethyl)-N-methylethylendiamin und Nitroaceton, beispielsweise, als Ausgangsstoffe eingesetzt werden, wird das Verfahren durch das folgende Reaktionsschema dargestellt:

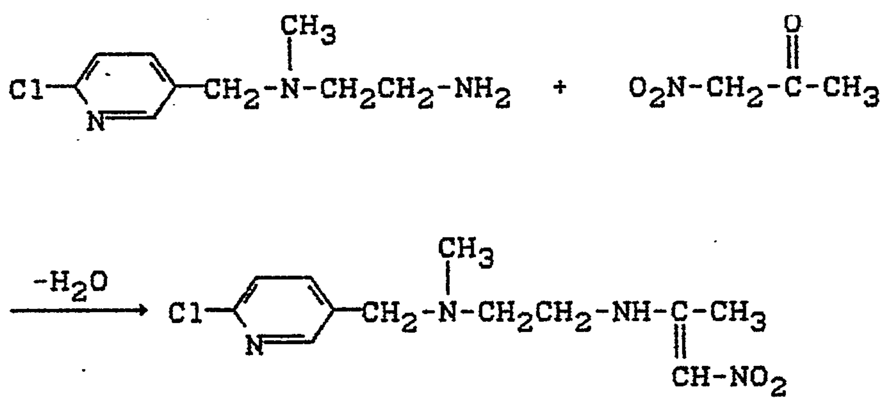


Wenn im Verfahren e) N-(2-Chloro-5-pyridylmethyl)-N'-(1-mercapto-2-nitrovinyl)ethylendiamin und -Chloro-5-chloromethylpyridin, beispielsweise, als Ausgangsstoffe eingesetzt werden, wird das Verfahren durch das folgende Reaktionsschema dargestellt:

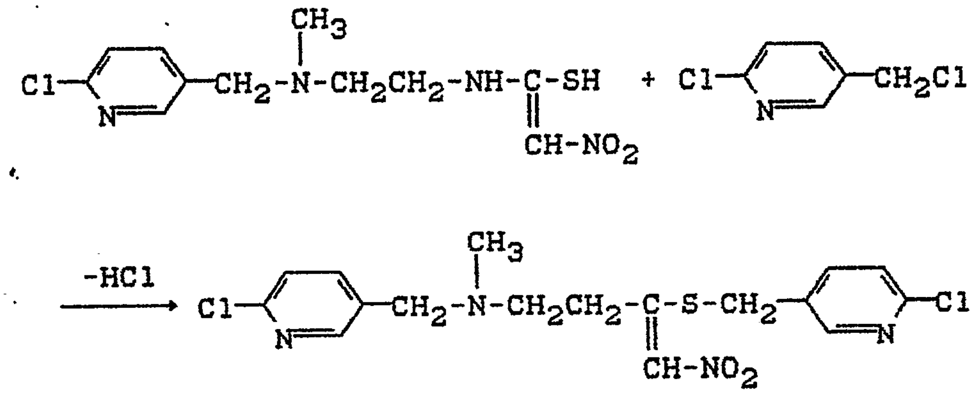

Unter den als Ausgangsstoffen in dem Verfahren a) eingesetzten Verbindungen der Formel (II) sind Verbindungen auf der Grundlage der obigen Definitionen für $R^1$, $R^2$, $R^3$, $W^1$ und A, vorzugsweise der obigen bevorzugten Definitionen, zu verstehen.

Die Verbindungen der Formel (II) umfassen sowohl neue als auch bekannte Verbindungen und können beispielsweise mittels des nachstehenden allgemeinen Verfahrens synthetisiert werden.

Verfahren f)

Ein Verfahren zur Herstellung der Verbindungen der Formel (II) umfaßt die Reaktion einer Verbindung der Formel

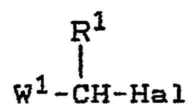

(X)

in der $R^1$, $W^1$ und Hal die im Vorstehenden angegebenen Bedeutungen haben mit einer Verbindung der Formel

$R^2$-NH-A-NH-$R^3$ (XI),

in der $R^2$, $R^3$ und A die im Vorstehenden angegebenen Bedeutungen haben.

Die Verbindungen der Formel (X) als Ausgangsstoffe in dem Verfahren f) sind bereits in den von der Anmelderin der vorliegenden Anmeldung eingereichten JP-Patentanmeldungen 26 020/1984 (JP-OS 172 976/1985), 72 966/1984 (JP-OS 218 386/1985), 132 943/1984 (JP-OS 12 682/1986), 18 627/1985, 18 628/1985, 23 683/1985, 106 853/1985 und 106 854/1985 offenbart.

Die Verbindungen der Formel (XI) als Ausgangsstoffe waren auf dem Gebiet der organischen Chemie vor der Einreichung der vorliegenden Anmeldung bekannt. Beispielsweise ist N-Methylethylendiamin aus J. Am. Chem. Soc. 73, Seiten 1370–1371 (1951), bekannt.

Das vorstehende Verfahren f) kann in einfacher Weise so durchgeführt werden, wie es in nachstehend angegebenen Beispielen aufgezeigt ist, und die als Ausgangsstoff in dem Verfahren a) erwünschte Verbindung der Formel (II) kann dabei erhalten werden.

Spezielle Beispiele für die Verbindungen der Formel (II) sind
N-(2-Chloro-5-pyridylmethyl)-N-methylethylendiamin,
N-(2-Chloro-5-pyridylmethyl)-N-ethylethylendiamin,
N-(2-Chloro-5-pyridylmethyl)ethylendiamin und
N-(2-Chloro-5-pyridylmethyl)trimethylendiamin.

In gleicher Weise sind in dem Verfahren a) als Ausgangsverbindungen der Formel (III) Verbindungen auf der Grundlage der Definitionen für $R^7$, Y und Z, vorzugsweise der obigen bevorzugten Definitionen für Y und Z und der bevorzugten Definition für $R^4$ als $R^7$, zu verstehen.

Die Verbindungen der Formel (III) umfassen bekannte Verbindungen, die bereits in den von der Anmelderin der vorliegenden Anmeldung eingereichten JP-Patentanmeldungen 219 082/1985 und 48 629/1986 beschrieben sind. Zu speziellen Beispielen zählen
1-Nitro-2,2-bis(methylthio)ethylen,
1-Nitro-2,2-bis(ethylthio)ethylen und
Dimethylcyanodithioimidcarbonat.

Die Verbindung der Formel (III) kann in dem Fall, in dem einer der beiden Substituenten $R^7$ Wasserstoff ist, durch die folgenden Grenzstrukturen bezeichnet werden.

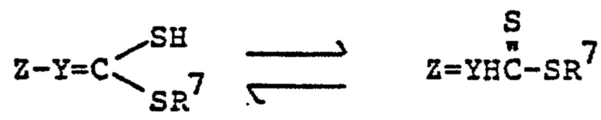

Speziell entspricht eine Verbindung mit der obigen rechten Formel in dem Fall, in dem Z Nitro ist und Y –CH= ist, genau einem Ester der Nitrodithioessigsäure, die eine bekannte, in Chem. Ber. 100, Seite 591, beschrieben Verbindung ist.

Unter den als Ausgangsstoffen in dem Verfahren b) eingesetzten Verbindungen der Formel (IV) sind Verbindungen auf der Grundlage der obigen Definitionen für $R^1$, $R^2$, $R^3$, Y, Z, $W^1$ und A, vorzugsweise der obigen bevorzugten Definitionen für $R^1$, $R^2$, $R^3$, Y, Z, $W^1$ und A zu verstehen.

Die meisten der Verbindungen der Formel (IV) werden von den Verbindungen der Formel (I) gemäß der vorliegenden Erfindung mitumfaßt, die mittels des oben angegebenen Verfahrens a) synthetisiert werden.

Unter den Verbindungen der Formel (V), die in gleicher Weise Ausgangsstoffe sind, sind Verbindungen auf der Grundlage der obigen Definitionen für $R^4$ und $R^5$, vorzugsweise der obigen bevorzugten Definitionen für $R^4$ und $R^5$, zu verstehen.

Die Verbindungen der Formel (V) sind auf dem Gebiet der organischen Chemie wohlbekannt.

Die als Ausgangsstoffe in dem Verfahren c) eingesetzten Verbindungen der Formel (II) sind mit den im vorstehenden Verfahren a) eingesetzten Verbindungen der Formel (II) synonym.

In gleicher Weise bezeichnen die Verbindungen der Formel (VI) als Ausgangsstoffe Verbindungen auf der Grundlage der obigen Definitionen für $R^4$, $R^5$ und Z, vorzugsweise der obigen bevorzugten Definitionen für $R^4$, $R^5$und Z.

Die Verbindungen der Formel (VII) sind auf dem Gebiet der organischen Chemie wohlbekannt, und zu speziellen Beispielen zählen Nitroguanidin und Cyanoguanidin. N-Substitutionsprodukte dieser Verbindungen sind ebenfalls bekannte Verbindungen, die beispielsweise in der US-PS 2 559 085, J. Am. Chem. Soc. 71, Seiten 1986–1970 (1949) und der GB-PS 599 722 beschrieben sind.

Die Verbindungen der Formel (II) als Ausgangsstsoffe in dem Verfahren d) sind mit den Verbindungen der Formel (II) in Verfahren a) synonym.

In gleicher Weise bezeichnen die als Ausgangsstoffe eingesetzten Verbindungen der Formel (VII) Verbindungen auf der Grundlage der obigen Definitionen für $R^6$, $R^7$ und Z, vorzugsweise der obigen bevorzugten Definitionen für $R^6$, $R^7$ und Z.

Die Verbindungen der Formel (VII) umfassen bekannte Verbindungen und sind beispielsweise beschrieben in Synthesis 1979, Seiten 295–296, und J. Org. Chem. 20, Seiten 927–936 (1955). Spezielle Beispiele sind 1-Nitro-2-propanon, 2-Nitroacetophenon und 3-Nitro-2-butanon.

Die Verbindungen der Formel (VIII) als Ausgangsstoffe in dem Verfahren e) bezeichnen Verbindungen auf der Grundlage der obigen Definitionen für $R^1$, $R^2$, $R^3$, Y, Z und $W^1$, vorzugsweise der obigen bevorzugten Definitionen für diese Symbole.

Die Verbindungen der Formel (VIII) werden von den durch die Formel (I) bezeichneten Verbindungen der vorliegenden Erfindung umfaßt, die mittels des Verfahrens a) hergestellt werden.

Die in gleicher Weise als Ausgangsstoffe eingesetzten Verbindungen der Formel (IX) bezeichnen Verbindungen auf der Grundlage der obigen Definitionen für $R^8$ und Hal, vorzugsweise der obigen bevorzugten Definitionen für $R^4$, das $R^8$ entspricht, und von Chloro und Bromo für Hal.

Spezielle Beispiele für die Verbindungen der Formel (IX) sind Benzylchlorid und 2-Chloro-5-chloromethylpyridin.

Für die praktische Durchführung des Verfahrens a) lassen sich sämtliche für die Umsetzung inerten organischen Lösungsmittel als geeignete Verdünnungsmittel anführen.

Zu Beispielen für solche Verdünnungsmittel zählen Wasser, aliphatische, alicyclische oder aromatische (gegebenfalls chlorierte) Kohlenwasserstoffe wie Hexan, Cyclohexan, Petrolether, Ligroin, Benzol, Toluol, Xylol, Methylenchlorid, Chloroform, Kohlenstofftetrachlorid, Ethylenchlorid, Trichloroethylen und Chlorbenzol; Ether wie Diethylether, Methylethylether, Di-isopropylether, Dibutylether, Propylenoxid, Dioxan und Tetrahydrofuran; Ketone wie Aceton, Methylethylketon, Methylisopropylketon und Methylisobutylketon; Nitrile wie Acetonitril, Propionitril und Acrylnitril; Alkohole wie Methanol, Ethanol, Isopropanol, Butanol und Ethylenglycol; Ester wie Ethylacetat und Amylacetat; Säureamide wie Dimethylformamid und Dimethylacetamid; Sulfone und Sulfoxide wie Dimethylsulfoxid und Sulfolan; und Basen wie Pyridin.

Das Verfahren a) kann in einem Temperaturbereich von beträchtlicher Breite durchgeführt werden, beispielswiese bei Temperaturen zwischen etwa 0°C und etwa 100°C, vorzugsweise zwischen etwa 20°C und etwa 80°C.

Die Reaktion wird zweckmäßigerweise unter Atmosphärendruck durchgeführt, jedoch ist es auch möglich, bei erhöhtem oder vermindertem Druck zu arbeiten.

Bei der praktischen Durchführung des Verfahrens a) können die gewünschten Verbindungen der Formel (I) dadurch erhalten werden, daß 1 mol der Verbindung der Formel (II) und 1 mol oder geringfügig

mehr als 1 mol der Verbindung der Formel (III), beispielsweise unter Rückfluß, in einem inerten Lösungsmittel erhitzt werden, bis die Entwicklung von Mercaptan aufhört.

Für die praktische Durchführung des Verfahrens b) können die gleichen für die Umsetzung inerten Lösungsmittel, wie sie im Vorstehenden für das Verfahren a) beispielhaft genannt wurden, angeführt werden.

Das Verfahren b) kann in einem weiten Temperaturbereich durchgeführt werden, beispielsweise bei einer Temperatur zwischen etwa 0°C und etwa 150°C, vorzugsweise zwischen etwa 20°C und etwa 90°C.

Vorzugsweise wird die Reaktion unter Atmosphärendruck durchgeführt, jedoch kann sie auch unter erhöhtem oder vermindertem Druck durchgeführt werden.

Bei der praktischen Durchführung des Verfahrens b) können die gewünschten Verbindungen der Formel (I) durch Erhitzen der Verbindung der Formel (IV) und der Verbindungen der Formel (V) unter Rückfluß in einem inerten Lösungsmittel in gleicher Weise wie im Verfahren a) erhalten werden.

Für die praktische Durchführung des Verfahrens c) können die gleichen inerten Lösungsmittel, wie sie im Vorstehenden für das Verfahren a) beispielhaft genannt wurden, als geeignete Verdünnungsmittel angeführt werden.

Das Verfahren c) kann in einem weiten Temperaturbereich durchgeführt werden, beispielsweise bei Temperaturen zwischen etwa 0°C und etwa 150°C, vorzugsweise zwischen etwa 20°C und etwa 90°C.

Vorzugsweise wird die Reaktion unter Atmosphärendruck durchgeführt, jedoch kann sie auch unter erhöhtem oder vermindertem Druck durchgeführt werden.

Bei der praktischen Durchführung des Verfahrens c) können die gewünschten Verbindungen der Formel (I) dadurch erhalten werden, daß 1 mol der Verbindung der Formel (II) und 1 mol oder geringfügig mehr als 1 mol der Verbindung der Formel (VI), beispielsweise in einem für die Umsetzung inerten Lösungsmittel und, entsprechend den Erfordernissen, unter sauren Bedingungen, umgesetzt werden.

Für die praktische Durchführung des Verfahrens d) können die gleichen inerten Lösungsmittel, wie sie im Vorstehenden für das Verfahren a) beispielhaft genannt wurden, als geeignete Verdünnungsmittel angeführt werden.

Das Verfahren d) kann in einem weiten Temperaturbereich durchgeführt werden, beispielsweise bei Temperaturen zwischen etwa 0°C und etwa 150°C, vorzugsweise zwischen etwa 20°C und etwa 100°C.

Vorzugsweise wird die Reaktion unter Atmosphärendruck durchgeführt, jedoch kann sie auch unter erhöhtem oder vermindertem Druck durchgeführt werden.

Bei der praktischen Durchführung des Verfahrens d) können die gewünschten Verbindungen der Formel (I) dadurch erhalten werden, daß 1 mol der Verbindungen der Formel (II) und 1 mol oder geringfügig mehr als 1 mol der Verbindungen der Formel (VII), beispielsweise unter Rückfluß in einem für die Umsetzung inerten Lösungsmittel erhitzt werden, wobei das Nebenprodukt Wasser entfernt wird.

Für die praktische Durchführung des Verfahrens e) können die gleichen inerten Lösungsmittel, wie sie im Vorstehenden für das Verfahren a) beispielhaft genannt wurden, als geeignete Verdünnungsmittel angeführt werden.

Das Verfahren e) kann in einem weiten Temperaturbereich durchgeführt werden, beispielsweise bei Temperaturen zwischen etwa 0°C und etwa 150°C, vorzugsweise zwischen etwa 20°C und etwa 80°C.

Vorzugsweise wird die Reaktion unter Atmosphärendruck durchgeführt, jedoch kann sie auch unter erhöhtem oder vermindertem Druck durchgeführt werden.

Bei der praktischen Durchführung des Verfahrens e) können die gewünschten Verbindungen der Formel (I) dadurch erhalten werden, daß 1 mol der Verbindungen der Formel (VIII) und 1 mol oder geringfügig mehr als 1 mol der Verbindungen der Formel (IX), beispielsweise in einem inerten Lösungsmittel, vorzugsweise in Gegenwart einer Base, umgesetzt werden.

In dem Fall, in dem die Gruppe $-X-R^4$ in den Verbindungen der Formel (I) gemäß der vorliegenden Erfindung -SH ist, können die Verbindungen durch die folgenden tautomeren Resonanzstrukturen bezeichnet werden.

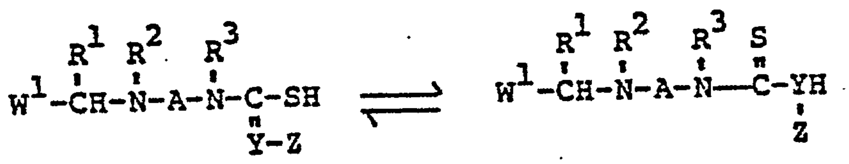

Weiterhin können in dem Fall, in dem die Gruppe $-X-R^4$ $-NHR^5$ ist, die Verbindungen der vorliegenden Erfindung durch die folgenden tautomeren Resonanzstrukturen bezeichnet werden.

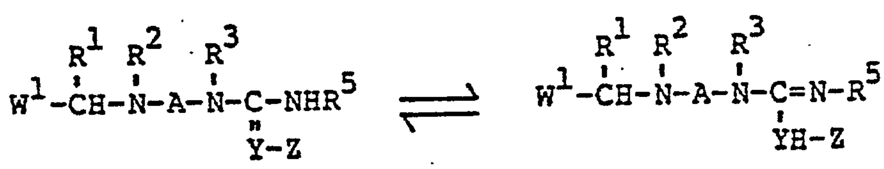

In dem Fall, in dem die Gruppe $R^3$ H ist, können die Verbindungen der vorliegenden Erfindung durch die folgenden tautomeren Resonanzstrukturen bezeichnet werden.

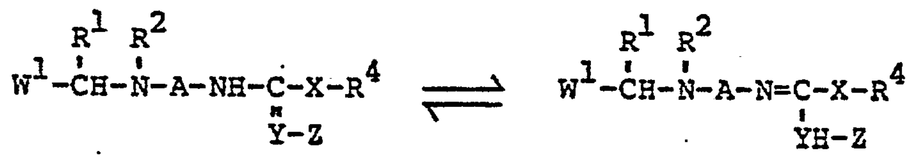

Die Wirkstoffe gemäß Formel (I) werden von Pflanzen gut vertragen, haben einen günstigen Wert der Toxizität gegenüber warmblütigen Tieren und lassen sich einsetzen zur Bekämpfung von Arthropoden-Schädlingen, insbesondere von Insekten, die in der Land- und Forstwirtschaft, bei Lagerprodukten und -materialien und auf dem Gebiet der Hygiene auftreten. Sie sind gegenüber normal empfindlichen und resistenten Species sowie gegen alle oder einige Entwicklungsstadien wirksam. Zu den oben genannten Schädlingen zählen:

Aus der Klasse der Isopoda beispielsweise
Oniscus asellus,
Armadillidium vulgare und
Porcellio scaber;
aus der Klasse der Diplopoda beispielsweise
Blaniulus guttulatus;
aus der Klasse der Chilopoda beispielsweise
Geophilus carpophagus und
Scutigera spec.;
aus der Klasse der Symphyla beispielsweise
Scutigerella immaculata;
aus der Ordnung der Thysanura beispielsweise
Lepisma saccharina;
aus der Ordnung der Collembola beispielsweise
Onychiurus armatus;
aus der Ordnung der Orthoptera beispielsweise
Blatta orientalis,
Periplaneta americana,
Leucophaea maderae,
Blatella germanica,
Acheta domesticus,
Gryllotalpa spp.,
Locusta migratoria migratorioides,
Melanoplus differentialis und
Schistocerca gregaria;
aus der Ordnung der Dermaptera beispielsweise
Forficula auricularia,
aus der Ordnung der Isoptera beispielsweise
Reticulitermes spp.;
aus der Ordnung der Anoplura beispielsweise
Phylloxera vastatrix,
Pemphigus spp.,
Pediculus humanus corporis,
Haematopinus spp. und
Linognathus spp.;
aus der Ordnung der Mallophaga beispielsweise
Trichodectes spp. und
Damalinea spp.;
aus der Ordnung der Thysanoptera beispielsweise
Hercinothrips femoralis und
Thrips tabaci;
aus der Ordnung der Heteroptera beispielsweise
Eurygaster spp.,
Dysdercus intermedius,
Piesma quadrata,
Cimex lectularius,
Rhodnius prolixus und
Triatoma spp.;

aus der Ordnung der Homoptera beispielsweise
- Aleurodes brassicae,
- Bemisia tabaci,
- Trialeurodes vaporariorum,
- Aphis gossypii,
- Brevicoryne brassicae,
- Cryptomyzus ribis,
- Aphis fabae,
- Doralis pomi,
- Eriosoma lanigerum
- Hyalopterus arundinis,
- Macrosiphum avenae,
- Myzus spp.,
- Phorodon humuli,
- Rhopalosiphum padi,
- Empoasca spp.,
- Euscelis bilobatus,
- Nephotettix cincticeps,
- Lecanium corni,
- Saissetia oleae,
- Laodelphax striatellus,
- Nilaparvata lugens,
- Aonidiella aurantii,
- Aspidiotus hederae,
- Pseudococcus sepp. und
- Psylla spp.;

aus der Ordnung der Lepidoptera beispielsweise
- Pectinophora gossypiella,
- Bupalus piniarius,
- Cheimatobia brumata,
- Lithocolletis blancardella,
- Hyponomeuta padella,
- Plutella maculipennis,
- Malacosoma neustria,
- Euproctis chrysorrhoea,
- Lymantria spp.,
- Bucculatrix thurberiella,
- Phyllocnistis citrella,
- Agrotis spp.,
- Euxoa spp.,
- Feltia spp.,
- Earias insulana,
- Heliothis spp.,
- Spodoptera exigua,
- Mamestra brassicae,
- Panolis flammea,
- Prodenia litura,
- Spodoptera spp.,
- Trichoplusia ni,
- Carpocapsa pomonella,
- Pieris spp.,
- Chilo spp.,
- Pyrausta nubilalis,
- Ephestia kuehniella,
- Galleria mellonella,
- Cacoecia podana,
- Capua reticulana,
- Choristoneura fumiferana,
- Clysia ambiguella,
- Homona magnanima und
- Tortrix viridana;

aus der Ordnung der Coleoptera beispielsweise
- Anobium punctatum,
- Rhizopertha dominica,
- Acanthoscelides obtectus,

Hylotrupes bajulus,
Agelastica alni,
Leptinotarsa decemlineata,
Phaedon cochleariae,
Diabrotica spp.,
Psylliodes chrysocephala,
Epilachna varivestis,
Atomaria spp.,
Oryzaephilus surinamensis,
Anthonomus spp.,
Sitophilus spp.,
Otiorrhynchus sulcatus,
Cosmopolites sordidus,
Ceuthorrhynchus assimilis,
Hypera postica,
Dermestes spp.,
Trogoderma spp.,
Anthrenus spp.,
Attagenus spp.,
Lyctus spp.,
Meligethes aeneus,
Ptinus spp.,
Niptus hololeucus,
Gibbium psylloides,
Tribolium spp.,
Tenebrio molitor,
Agriotes spp.,
Conoderus spp.,
Melolontha melolontha,
Amphimallon solstitialis und
Costelytra zealandica;

aus der Ordnung der Hymenoptera beispielsweise
Diprion spp.,
Hoplocampa spp.,
Lasius spp.,
Monomorium pharaonis und
Vespa spp.;

aus der Ordnung der Diptera beispielsweise
Aedes spp.,
Anopheles spp.,
Culex spp.,
Drosophila melanogaster,
Musca spp.,
Fannia spp.,
Calliphora erythrocephala,
Lucilia spp.,
Chrysomyia spp.,
Cuterebra spp.,
Gastrophilus spp.,
Hyppobosca spp.,
Stomoxys spp.,
Oestrus spp.,
Hypoderma spp.,
Tabanus spp.,
Tannia spp.,
Bibio hortulanus,
Oscinella frit,
Phorbia spp.,
Pegomyia hyoscami,
Ceratitis capitata,
Dacus oleae und
Tipula paludosa.

Auf dem Gebiet der Veterinärmedizin sind die neuen Verbindungen der vorliegenden Erfindung gegen verschiedene schädliche Tierparasiten (innere und äußere Parasiten) wie Insekten und Würmer wirksam.

Beispielen für solche Tierparasiten sind Insekten wie
Gastrophilus spp.,
Stomoxys spp.,
Trichodectes spp.,
Rhodnius spp. und
Ctenocephalidex canis.

Die Aktiven Verbindungen können in gebräuchliche Formulierungen überführt werden, etwa Lösungen, Emulsionen, Suspensionen, Pulver, Schäume, Pasten, Granulat, Aerosole, mit dem Wirkstoff der Formel (I) imprägnierte natürliche oder synthetische Stoffe, sehr feine Kapseln in polymeren Substanzen, Überzugsmassen zur Verwendung auf Saatgut (Beizmittel) sowie Formulierungen für den Einsatz mit Verbrennungseinrichtungen wie Räucherpatronen, Räucherdosen und Räucherschlangen sowie für die kalte Vernebelung und die warme Vernebelung nach dem Ultra-Low-Volume-Verfahren.

Diese Formulierungen können in bekannter Weise hergestellt werden, beispielsweise durch Vermischen der Wirkstoff mit Streckmitteln, das heißt mit flüssigen oder verflüssigten gasförmigen oder festen Verdünnungsmitteln oder Trägern, gegebenenfalls unter Verwendung grenzflächenaktiver Mittel, das heißt von Emulgatoren und/oder Dispergiermitteln und/oder schaumbildenden Mitteln. Bei Verwendung von Wasser als Streckmittel können organische Lösungsmittel beispielsweise als Hilfslösungsmittel verwendet werden.

Als flüssige Lösungsmittel, Verdünnungsmittel oder Träger vorzugsweise geeignet sind aromatische Kohlenwasserstoffe wie Xylol, Toluol oder Alkylnaphthaline, chlorierte aromatische oder chlorierte aliphatische Kohlenwasserstoffe wie Chlorbenzole, Chloroethylene oder Methylenchlorid, aliphatische Kohlenwasserstoffe wie Cyclohexan oder Paraffine, beispielsweise Mineralöl-Fraktionen, Alkohole wie Butanol oder Glycol sowie deren Ether und Ester, Ketone wie Aceton, Methylethylketon, Methylisobutylketon oder Cyclohexanon oder stark polare Lösungsmittel wie Dimethylformamid und Dimethylsulfoxid sowie auch Wasser.

Unter verflüssigten gasförmigen Verdünnungsmitteln oder Trägern sind Flüssigkeiten zu verstehen, die bei normaler Temperatur und normalem Druck gasförmig wären, beispielsweise Aerosol-Treibmittel wie halogenierte Kohlenwasserstoffe sowie Butan, Propan, Stickstoff und Kohlenstoffdioxid.

Als feste Träger verwendbar sind gemahlene natürliche Minerale wie Kaoline, Tone, Talkum, Kreide, Quarz, Attapulgit, Montmorillonit oder Diatomeenerde und gemahlene synthetische Minerale wie hochdisperse Kieselsäure, Aluminiumoxid und Silicate. Als feste Träger für Granulat können zerkleinerte und fraktionierte Natursteinmaterialien verwendet werden, etwa Calcit, Marmor, Bimsstein, Sepiolith und Dolomit sowie synthetisches Granulat aus anorganischen und organischen Mehlen und Granulat aus organischem Material wie Sägemehl, Kokosnußschalen, Maiskolben und Tabakstengel.

Als emulgierende und/oder schaumbildende Mittel können nicht-ionische und anionische Emulgatoren wie Polyoxyethylenfettsäureester, Polyoxyethylenfettalkoholether, beispielsweise Alkylarylpolyglycol-Ether, Alkylsulfonate, Alkylsulfate, Arylsulfonate sowie Albumin-Hydrolyseprodukte verwendet werden. Zu Dispergiermitteln zählen beispielsweise Ligninsulfit-Ablaugen und Methylcellulose.

Haftmittel wie Carboxymethylcellulose und natürliche und synthetische Polymere in Form von Pulvern, Granulat oder Latices wie Gummi arabicum, Polyvinylalkohol und Polyvinylacetat können bei der Formulierung verwendet werden.

Es ist möglich, bei der Herstellung der Formulierungen farbgebende Mittel, etwa anorganische Pigmente wie beispielsweise Eisenoxid, Titanoxid und Preußisch blau und organische Farbstoffe wie Alizarin-Farbstoffe, Azo-Farbstoffe oder Metallphthalocyanin-Farbstoffe, sowie Spuren-Nährstoffe wie Salze von Eisen, Mangan, Bor, Kupfer, Cobalt, Molybdän und Zink zu verwenden.

Die Formulierungen enthalten im allgemeinen 0,1 bis 95 Gew.-%, vorzugsweise 0,5 bis 90 Gew.-%, des Wirkstoffs der allgemeinen Formel (I).

Die erfindungsgemäßen Wirkstoffe der Formel (I) können in ihren handelsüblichen Formulierungen sowie den aus diesen Formulierungen hergestellten Anwendungsformen im Gemisch mit anderen Wirkstoffen vorliegen, etwa mit Insektiziden, Ködern, Sterilisationsmitteln, Akariziden, Nematiziden, Fungiziden, Wachstumsregulatoren oder Herbiziden. Zu den Insektiziden gehören beispielsweise Phosphate, Carbamate, Carboxylate, chlorierte Kohlenwasserstoffe, Phenylharnstoffe und von Mikroorganismen erzeugte Substanzen.

Die erfindungsgemäßen Wirkstoffe der Formel (I) können weiterhin in ihren handelsüblichen Formulierungen oder den aus diesen Formulierungen hergestellten Anwendungsformen im Gemisch mit synergistischen Mitteln vorliegen. Synergistische Mittel sind Verbindungen die die Wirkung der aktiven Verbindungen steigern, ohne daß es für das zugesetzte synergistische Mittel erforderlich ist, selbst aktiv zu sein.

Der Gehalt des Wirkstoffs in den aus den im Handel erhältlichen Formulierungen hergestellten Anwendungsformen kann innerhalb weiter Grenzen variieren.

Die Konzentration des Wirkstoffs in den Anwendungsformen kann 0,0000001 bis 100 Gew.-% betragen und liegt vorzugsweise zwischen 0,0001 und 1 Gew.-%.

Die Verbindungen werden in üblicher Weise in einer den Anwendungsformen angemessenen Form zur Anwendung gebracht.

Bei der Verwendung gegen Hygiene-Schädlinge und Schädlinge in Produkt-Vorräten zeichnen sich die Wirkstoffe durch eine hervorragende Residualwirkung auf Holz und Ton sowie eine gute Beständigkeit gegen Alkali auf gekalkten Unterlagen aus.

Die Herstellung und Verwendung der erfindungsgemäßen Wirkstoffe läßt sich beispielhaft mit Hilfe der folgenden Beispiele erläutern, soll aber keinesfalls auf diese Beispiele beschränkt bleiben.

Herstellungsbeispiele

Beispiel 1

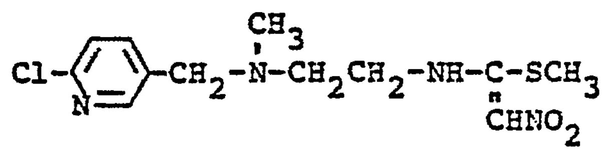

(Verbindung Nr. 4)

Eine Mischung von N-(2-Chloro-5-pyridylmethyl)-N-methylethylendiamin (2,0 g), 1-Nitro-2,2-bis(methylthio)ethylen (1,7 g) und Ethanol (40 ml) wurde 5 h unter Rühren zum Rückfluß erhitzt. Die Reaktionsmischung wurde auf etwa die Hälfte ihres Volumens eingeengt und gekühlt. Die ausgefallenen Kristalle wurden durch Filtration gesammelt, mit einer kleinen Menge Ethanol gewaschen und getrocknet, wonach das gewünschte N-(2-Chloro-5-pyridylmethyl)-N-methyl-N′-(1-methylthio-2-nitrovinyl)ethylendiamin (1,8 g) mit einem Schmp. 93–95°C erhalten wurde.

Beispiel 2

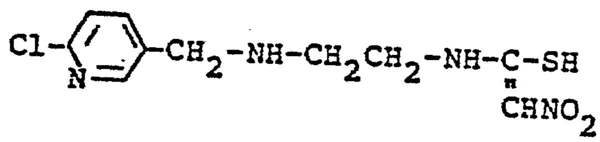

(Verbindung Nr. 2)

N-(2-Chloro-5-pyridylmethyl)ethylendiamin (2,0 g) und Methyl-2-nitrodithioacetat (1,7 g) wurden in Methanol (40 ml) gelöst, und die Lösung wurde 1 h im Stickstoff-Strom auf 50°C erhitzt. Die Lösung wurde auf Raumtemperatur gekühlt, worauf sich Kristalle abschieden. Die Kristalle wurden durch Filtration gesammelt, mit einer kleinen Menge Methanol gewaschen und dann getrocknet, wonach das gewünschte N-(2-Chloro-5-pyridylmethyl)-N′-(1-mercapto-2-nitrovinyl)ethylendiamin (1,2 g) mit einem Schmp. 136–137°C (Zers.) erhalten wurde.

Beispiel 3

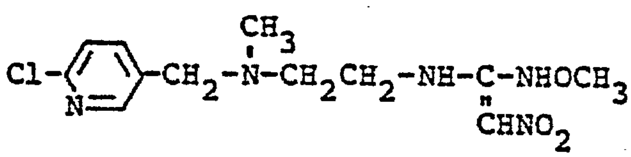

(Verbindung Nr. 26)

Zu einer Lösung von 0,95 g der im vorstehenden Beispiel 1 synthetisierten Verbindung in 30 ml Methanol wurde O-Methylhydroxylamin (0,16 g) hinzugefügt, und die Mischung wurde 1 h unter Rückfluß erhitzt. Das Ethanol wurde unter vermindertem Druck abgedampft, und der Rückstand wurde durch Silicagel-Säulenchromatographie gereinigt, wonach das gewünschte N-(2-Chloro-5-pyridylmethyl)-N-methyl-N′-(1-methoxyamino-2-nitrovinyl)ethylendiamin (0,6 g) mit einem $n_D^{20}$ von 1,5466 erhalten wurde.

Beispiel 4

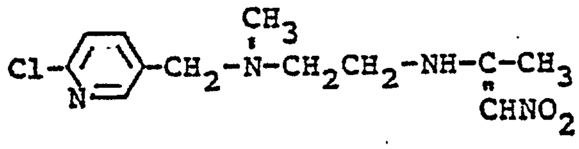

(Verbindung Nr. 20)

Nitroaceton (1,1 g) und N-(2-Chloro-5-pyridylmethyl)-N-methylethylendiamin (2,0 g) wurden in Toluol (60 ml) gelöst, und die Lösung wurde 3 h in einem mit einer Wasser-Abtrennvorrichtung ausgerüsteten Kolben zum Rückfluß erhitzt, wobei das durch die Reaktion gebildete Wasser entfernt wurde. Die Toluol-Lösung wurde unter vermindertem Druck konzentriert, und der verbleibende feste Stoff wurde aus Ethanol umkristallisiert, wonach das gewünschte N-(2-Chloro-5-pyridylmethyl)-N-methyl-N'-(1-methyl-2-nitrovinyl)ethylendiamin (2,0 g) mit einem Schmp. 78–80°C erhalten wurde.

Beispiel 5

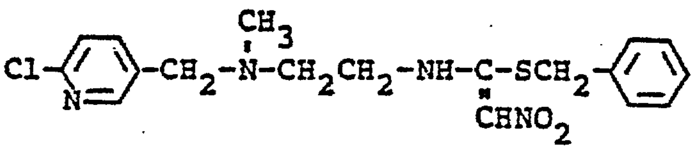

(Verbindung Nr. 12)

Die in Beispiel 2 synthetisierte Verbindung (1,4 g) wurde in trockenem Acetonitril (30 ml) gelöst, und Kaliumcarbonat (1,4 g) wurde hinzugefügt. Die Mischung wurde 30 min bei Raumtemperatur gerührt. Dann wurde Benzylbromid (0,9 g) zugegeben, und die Mischung wurde 2 h unter Rühren auf 50°C erhitzt. Die Mischung wurde auf Raumtemperatur gekühlt und zur Neutralisation in Wasser gegossen. Die wäßrige Schicht wurde mit Dichloromethan extrahiert. Das Rohprodukt wurde durch Silicagel-Säulenchromatographie gereinigt, wonach das gewünschte N-(2-Chloro-5-pyridylmethyl)-N-methyl-N'-(1-benzylthio-2-nitrovinyl)ethylendiamin (0,3 g) mit einem Schmp. 119-123°C erhalten wurde.

Verbindungen der Formel (I) gemäß der vorliegenden Erfindung, die nach den gleichen Methoden wie in den Beispielen 1 bis 5 hergestellt wurden, sind zusammen mit den in den Beispielen 1 bis 5 erhaltenen Verbindungen in der folgenden Tabelle 1 aufgeführt.

<u>Tabelle 1</u>

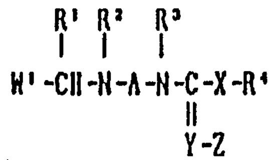

| Verb. Nr. | $W^1$ | $R^1$ | $R^2$ | A | $R^3$ | Y-Z | X | $R^4$ | Physikal. Konstante |
|---|---|---|---|---|---|---|---|---|---|
| 1 | 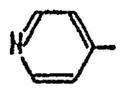 | H | $-CH_3$ | $-CH_2CH_2-$ | H | $=CH-NO_2$ | S | $-CH_3$ | Schmp. 126 ~ 129°C |
| 2 | 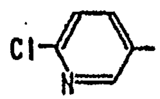 | H | H | $-CH_2CH_2-$ | H | $=CH-NO_2$ | S | H | Schmp. 136 ~ 137°C (Zers.) |
| 3 | 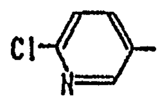 | H | H | $-CH_2CH_2CH_2-$ | H | $=CH-NO_2$ | S | H | Schmp. 131 ~ 135°C (Zers.) |
| 4 | 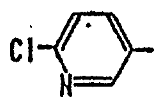 | H | $-CH_3$ | $-CH_2CH_2-$ | H | $=CH-NO_2$ | S | $-CH_3$ | Schmp. 93 ~ 95°C |
| 5 | 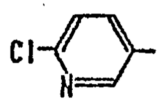 | H | $-CH_3$ | $-CH_2CH_2CH_2-$ | H | $=CH-NO_2$ | S | $-CH_3$ | |
| 6 | 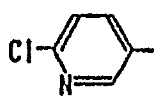 | H | $-C_2H_5$ | $-CH_2CH_2-$ | H | $=CH-NO_2$ | S | $-CH_3$ | |
| 7 | 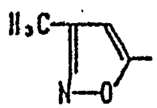 | H | $-CH_3$ | $-CH_2CH_2-$ | H | $=CH-NO_2$ | S | $-CH_3$ | Schmp. 81 ~ 84°C |

Tabelle 1 - Fortsetzung

| Verb. Nr. | W¹ | R¹ | R² | A | R³ | Y-Z | X | R⁴ | Physikal. Konstante |
|---|---|---|---|---|---|---|---|---|---|
| 8 | 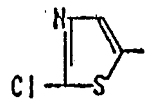 | H | $-CH_3$ | $-CH_2CH_2CH_2-$ | H | $=CH-NO_2$ | S | $-CH_3$ | Schmp. 113 ~ 116 °C |
| 9 | 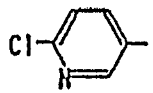 | H | $-CH_3$ | $-CH_2CH_2-$ | H | $=CH-NO_2$ | S | $-C_2H_5$ | Schmp. 65 ~ 67 °C |
| 10 | 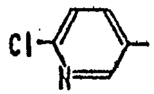 | H | $-CH_3$ | $-CH_2CH_2-$ | H | $=CH-NO_2$ | S | $-C_3H_7$-n | $n_D^{20}$ 1.6055 |
| 11 | 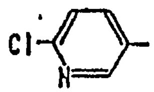 | H | $-CH_3$ | $-CH_2CH_2-$ | H | $=CH-NO_2$ | S | $-C_3H_7$-iso | $n_D^{20}$ 1.6080 |
| 12 | 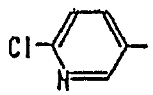 | H | $-CH_3$ | $-CH_2CH_2-$ | H | $=CH-NO_2$ | S | $-CH_2-$ 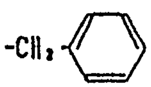 | Schmp. 119 ~ 123 °C |
| 13 | 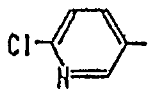 | H | $-C_2H_5$ | $-CH_2CH_2-$ | H | $=CH-NO_2$ | S | $-CH_2-$ 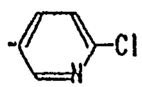 | |
| 14 | 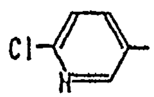 | H | $-CH_3$ | $-CH_2CH_2-$ | $-CH_3$ | $=CH-NO_2$ | S | $-CH_3$ | $n_D^{20}$ 1.6273 |
| 15 | 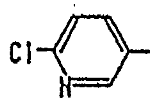 | H | $-CH_3$ | $-CH_2CH_2-$ | H | $=CH-NO_2$ | NH | H | Schmp. 120 ~ 123 °C |
| 16 | 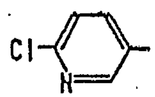 | H | $-CH_3$ | $-CH_2CH_2-$ | H | $=CH-NO_2$ | NH | $-CH_3$ | Schmp. 126 ~ 128 °C |

Tabelle 1 - Fortsetzung

| Verb. Nr. | W' | $R^1$ | $R^2$ | A | $R^3$ | Y-Z | X | $R^4$ | Physikal. Konstante |
|---|---|---|---|---|---|---|---|---|---|
| 17 | 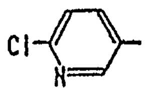 | H | $-CH_3$ | $-CH_2CH_2-$ | H | $=CH-NO_2$ | $N-CH_3$ | $-CH_3$ | Schmp. 79 ~ 86 °C |
| 18 | 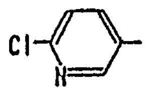 | H | $-CH_3$ | $-CH_2CH_2-$ | H | $=CH-NO_2$ | NH | $-N(CH_3)_2$ | |
| 19 | 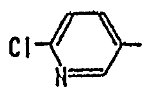 | H | $-CH_3$ | $-CH_2CH_2-$ | H | $=CH-NO_2$ | NH | 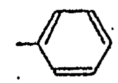 | Schmp. 179 ~ 182 °C |
| 20 | 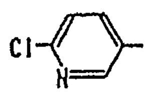 | H | $-CH_3$ | $-CH_2CH_2-$ | H | $=CH-NO_2$ | — | $-CH_3$ | Schmp. 78 ~ 80 °C |
| 21 | 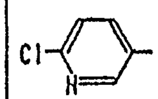 | H | $-CH_3$ | $-CH_2CH_2-$ | H | $=CH-NO_2$ | — | 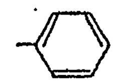 | $n_D^{19}$ 1.5685 |
| 22 | 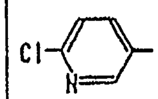 | H | $-C_2H_5$ | $-CH_2CH_2-$ | H | $=N-CN$ | S | $-CH_3$ | $n_D^{25}$ 1.5938 |
| 23 | 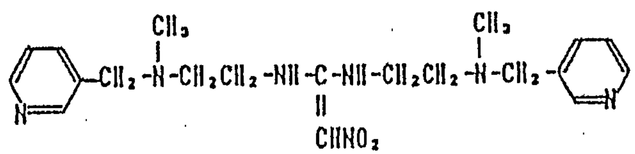 | | | | | | | | |
| 24 | 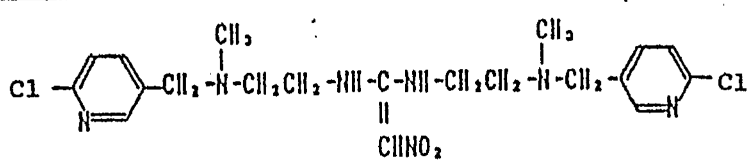 | | | | | | | | mp. 124~127 °C |

Tabelle 1 - Fortsetzung

| Verb. Nr. | W' | R' | R² | A | R³ | Y-Z | X | R⁴ | Physikal. Konstante |
|---|---|---|---|---|---|---|---|---|---|
| 25 | 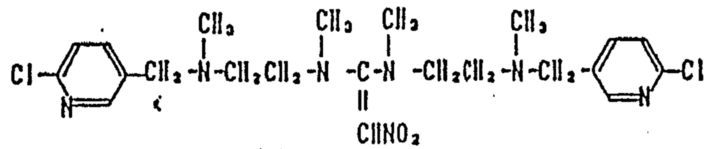 | | | | | | | | $n_D^{20}$ 1.5733 |
| 26 | 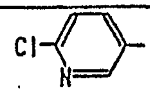 | H | $-CH_3$ | $-CH_2CH_2-$ | H | $=CH-NO_2$ | NH | $-OCH_3$ | $n_D^{20}$ 1.5466 |
| 27 | 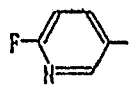 | H | $-CH_3$ | $-CH_2CH_2-$ | H | $=CH-NO_2$ | S | $-CH_3$ | |
| 28 | 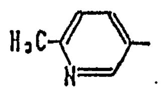 | H | $-CH_3$ | $-CH_2CH_2-$ | H | $=CH-NO_2$ | S | $-CH_2CH_2OC_2H_5$ | |
| 29 | 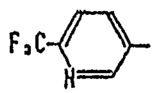 | H | $-CH_3$ | $-CH_2CH_2-$ | H | $=CH-NO_2$ | S | $-CH_2CH_2SC_2H_5$ | |
| 30 | 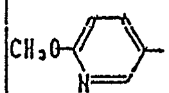 | H | $-CH_3$ | $-CH_2CH_2-$ | H | $=CH-NO_2$ | S | $-CH_3$ | |
| 31 | 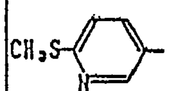 | H | $-CH_3$ | $-CH_2CH_2-$ | H | $=CH-NO_2$ | S | $-CH_3$ | |
| 32 | 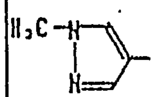 | H | $-CH_3$ | $-CH_2CH_2CH_2-$ | H | $=CH-NO_2$ | S | $-CH_3$ | |
| 33 | 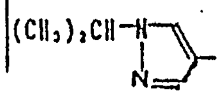 | H | $-CH_3$ | $-CH_2CH_2-$ | H | $=CH-NO_2$ | S | $-CH_3$ | |

**Tabelle 1 - Fortsetzung**

| Verb. Nr. | W¹ | R¹ | R² | A | R³ | Y-Z | X | R⁴ | Physikal. Konstante |
|---|---|---|---|---|---|---|---|---|---|
| 34 | 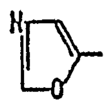 | $-CH_3$ | $-CH_3$ | $-CH_2CH_2-$ | H | $=CH-NO_2$ | S | $-CH_3$ | |
| 35 | 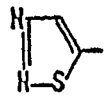 | H | $-C_2H_5$ | $-CH_2CH_2-$ | H | $=CH-NO_2$ | S | $-CH_3$ | |
| 36 | 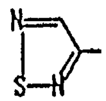 | H | $-CH_3$ | $-CH_2CH_2-$ | H | $=CH-NO_2$ | S | $-CH_3$ | |
| 37 | 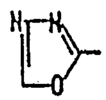 | H | $-CH_3$ | $-CH_2CH_2-$ | H | $=CH-NO_2$ | S | $-CH_3$ | |
| 38 | 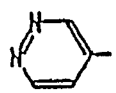 | H | $-CH_3$ | $-CH_2CH_2-$ | H | $=CH-NO_2$ | S | $-CH_3$ | |
| 39 | 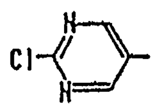 | H | $-CH_3$ | $-CH_2CH_2-$ | H | $=CH-NO_2$ | S | $-CH_3$ | |
| 40 | 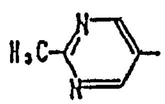 | H | $-CH_3$ | $-CH_2CH_2-$ | $-CH_3$ | $=CH-NO_2$ | S | $-CH_3$ | |
| 41 | 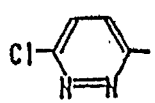 | H | $-CH_3$ | $-CH_2CH_2-$ | H | $=CH-NO_2$ | S | $-CH_3$ | |
| 42 | 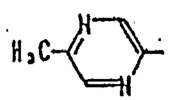 | H | $-CH_3$ | $-CH_2CH_2-$ | H | $=CH-NO_2$ | S | $-CH_3$ | |

Tabelle 1 - Fortsetzung

| Verb. Nr. | W' | R' | R² | A | R³ | Y-Z | X | R⁴ | Physikal. Konstante |
|---|---|---|---|---|---|---|---|---|---|
| 43 | 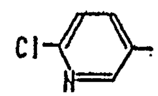 | H | $-CH_3$ | $-CH_2\overset{CH_3}{\underset{CH_3}{C}}-CH_2-$ | H | $=CH-NO_2$ | S | $-CH_3$ | |
| 44 | 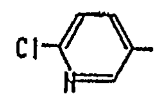 | $-CH_3$ | $-CH_3$ | $-CH_2CH_2-$ | H | $=CH-NO_2$ | S | $-CH_3$ | |
| 45 | 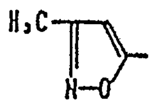 | H | $-CH_3$ | $-CH_2CH_2-$ | $-CH_3$ | $=CH-NO_2$ | NH | H | |
| 46 | 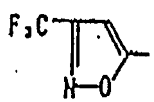 | H | $-CH_3$ | $-CH_2CH_2-$ | H | $=CH-NO_2$ | NH | $-OCH_3$ | |
| 47 | 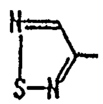 | H | $-CH_3$ | $-CH_2CH_2-$ | H | $=CH-NO_2$ | NH | $-C_3H_7$-iso | |
| 48 | 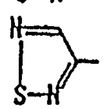 | H | $-CH_3$ | $-CH_2CH_2CH_2-$ | H | $=CH-NO_2$ | NH | $-N(CH_3)_2$ | |
| 49 | 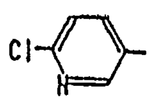 | H | $-CH_3$ | $-CH_2CH_2-$ | H | $=N-NO_2$ | NH | H | |
| 50 | 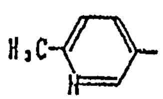 | H | $-CH_3$ | $-CH_2CH_2-$ | H | $=N-NO_2$ | NH | H | |
| 51 | 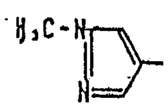 | H | $-CH_3$ | $-CH_2CH_2-$ | H | $=N-NO_2$ | NH | H | |

Tabelle 1 - Fortsetzung

| Verb. Nr. | W¹ | R¹ | R² | A | R³ | Y-Z | X | R⁴ | Physikal. Konstante |
|---|---|---|---|---|---|---|---|---|---|
| 52 | 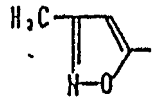 | H | $-CH_3$ | $-CH_2CH_2-$ | H | $=N-NO_2$ | NH | H | |
| 53 | 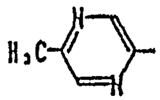 | H | $-CH_3$ | $-CH_2CH_2-$ | H | $=N-NO_2$ | NH | H | |
| 54 | 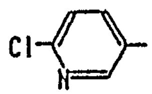 | H | $-CH_3$ | $-CH_2CH_2-$ | H | CN<br>$=C-CN$ | S | $-CH_3$ | |
| 55 | 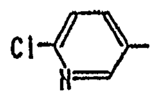 | H | $-CH_3$ | $-CH_2CH_2-$ | H | $COOC_2H_5$<br>$=C-CN$ | S | $-CH_3$ | |
| 56 | 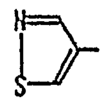 | H | $-CH_3$ | $-CH_2CH_2-$ | H | CN<br>$=C-CN$ | S | $-C_2H_5$ | |
| 57 | 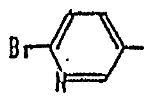 | H | $-CH_3$ | $-CH_2CH_2-$ | H | $=N-CN$ | S | $-CH_3$ | |
| 58 | 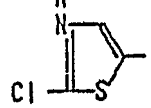 | H | $-CH_3$ | $-CH_2CH_2-$ | H | $=N-CN$ | S | $-CH_3$ | |
| 59 | 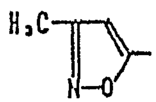 | H | $-CH_3$ | $-CH_2CH_2-$ | H | $=N-CN$ | S | $-CH_3$ | |
| 60 | 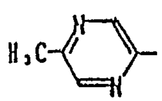 | H | $-CH_3$ | $-CH_2CH_2-$ | H | $=N-CN$ | S | $-CH_3$ | |

Beispiel 6

Synthese eines Ausgangsstoffs:

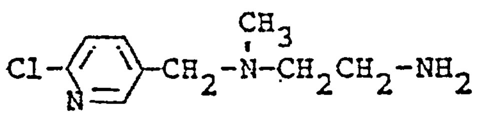

Eine Lösung von 2-Chloro-5-chloromethylpyridin (3,2 g) in 30 ml Acetonitril wurde tropfenweise unter gutem Rühren bei 5°C bis 10°C zu einer Lösung von N-Methylethylendiamin (7,4 g) in Acetonitril (50 ml) hinzugefügt. Nach der Zugabe wurde die Mischung 2 h bei Raumtemperatur gerührt. Aus der Mischung wurden zuerst das Acetonitril und dann der Überschuß an N-Methylethylendiamin unter vermindertem Druck abgedampft (wobei die Temperatur der Mischung unter 50°C gehalten wurde). Dem Rückstand wurde Dichloromethan zugesetzt, und die Mischung wurde zweimal mit einer kleinen Menge Wasser gewaschen und dann getrocknet. Abdampfen des Dichloromethans lieferte das gewünschte N-(2-Chloro-5-pyridylmethyl)-N-methylethylendiamin (2,7 g) als farbloses Öl mit einem $n_D^{20}$ von 1,5584.

Biologische Tests

Beispiel 7

Test mit gegen Organophosphor-Mittel resistenten Nephotettix cincticeps:

| Herstellung einer Test-Chemikalie: | | |
|---|---|---|
| Lösungsmittel: | Xylol | 3 Gew.-Teile |
| Emulgator: | Polyoxyethylen-alkylphenylether | 1 Gew.-Teil |

Zur Herstellung einer Formulierung eines geeigneten Wirkstoffs 1 Gew.-Teil des Wirkstoffs mit der oben bezeichneten Menge Lösungsmittel, das die oben angegebene Menge Emulgator enthielt, vermischt. Die Mischung wurde mit Wasser auf eine vorher festgesetzte Konzentration verdünnt.

Test-Verfahren:

Eine Wasser-Verdünnung jedes Wirkstoffes mit einer vorher festgelegten Konzentration, die wie oben beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, und über jeden der Töpfe wurde ein Drahtkorb von 7 cm Durchmesser und einer Höhe von 14 cm gestülpt, und 30 ausgewachsene weibliche Exemplare von Nephotettix cincticeps, die gegen Organophosphat-Chemikalien Resistenz zeigten, wurden unter dem Drahtkorb ausgesetzt. Die Töpfe wurden jeweils in einen Raum mit konstanter Temperatur gestellt, und zwei Tage später wurde die Zahl der toten Insekten bestimmt, und die Abtötungsrate wurde berechnet.

In diesem Test zeigten beispielsweise die Verbindungen Nr. 1, 2, 3, 4, 5, 6, 7, 10, 11, 12, 13, 15, 16, 18, 19, 20, 22, 24 und 25 eine Abtötungsrate von 100% bei einer Wirkstoff-Konzentration von 200 ppm.

Beispiel 8

Test mit Laternenträgern

Test-Verfahren:

Eine Wasser-Verdünnung jeder der aktiven Verbindungen mit einer vorher festgelegten Konzentration, die wie in Beispiel 7 beschrieben hergestellt worden war, wurde auf Reispflanzen von etwa 10 cm Höhe, die jeweils in Töpfen von 12 cm Durchmesser gezogen worden waren, in einer Menge von 10 ml pro Topf gesprüht. Die aufgesprühte Chemikalie wurde trocknen gelassen, und über jeden der Töpfe wurde ein Drahtkorb von 7 cm Durchmesser und 14 cm Höhe gestülpt. 30 ausgewachsene weibliche Exemplare von Nilaparvata lugens Stal eines Stammes, der gegen Organophosphat-Chemikalien Resistenz zeigte, wurden unter dem Korb ausgesetzt. Die Töpfe wurden in einem Raum mit konstanter Temperatur aufbewahrt, und zwei Tage später wurde die Zahl der toten Insekten bestimmt. Danach wurde die Abtötungsrate berechnet.

In der gleichen Weise wurde die Abtötungsrate für Sogatella furcifera Horvath und organophosphorresistente Laodelphax striatellus Fallen berechnet.

In diesem Test zeigten beispielsweise die Verbindungen Nr. 1, 2, 3, 4, 6, 7, 9, 10, 11, 12, 13, 17, 18, 19 und 26 eine Abtötungsrate von 100% bei einer Wirkstoff-Konzentration von 200 ppm gegen N. lugens, S. furcifera und L. striatellus.

Beispiel 9

Test mit Myzus persicae (grünen Pfirsichblattläusen), die gegen Organophosphat- und Carbamat-Chemikalien Resistenz zeigten:

Test-Verfahren:

Gezüchtete grüne Pfirsichblattläuse, die gegen Organophosphate und Carbamate Resistenz zeigten, wurden auf Auberginen-Setzlingen (schwarze längliche Auberginen) von etwa 20 cm Höhe ausgesetzt, die in unglasierten Töpfen von 15 cm Durchmesser gezogen worden waren (etwa 200 Blattläuse pro Setzling). Einen Tag nach dem Aussetzen wurde eine Wasser-Verdünnung jeder der aktiven Verbindungen mit einer vorher festgelegten Konzentration, die wie in Beispiel 7 hergestellt worden war, in genügenden Mengen mit Hilfe einer Spritzpistole auf die Pflanzen gesprüht. Nach dem Sprühen wurden die Töpfe in einem Gewächshaus bei 28°C stehen gelassen. 24 h nach dem Sprühen wurde die Abtötungsrate berechnet. Für jede Verbindung wurde der Test mit zwei Wiederholungen durchgeführt.

In diesem Test zeigten beispielsweise die Verbindungen Nr. 2, 4, 6, 7, 9, 10 und 12 eine Abtötungsrate von 100% bei einer Wirkstoff-Konzentration von 200 ppm.

**Patentansprüche**

1. Alkylendiamine der Formel (I)

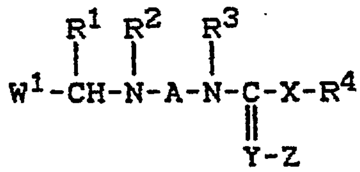

(I),

in welcher

$R^1$, $R^2$ und $R^3$ Wasserstoff oder $C_{1-4}$-Alkyl bezeichnen,

$R^4$ Wasserstoff, $C_{1-4}$-Alkyl, $C_{6-10}$-Ayl, Benzyl, Phenethyl, $C_{1-4}$-Alkoxy, Dialkylamino mit insgesamt 2 bis 6-Kohlenstoff-Atomen, Alkoxyalkyl mit insgesamt 2 bis 6 Kohlenstoff-Atomen, Alkylthioalkyl mit insgesamt 2 bis 4 Kohlenstoff-Atomen oder eine Gruppe der Formel $-CH_2-W^2$ bezeichnet, in der $W^2$ die gleichen Bedeutungen hat, wie sie hiernach für $W^1$ angegeben sind,

X S oder

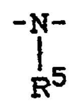

bezeichnen, worin $R^5$

Wasserstoff oder $C_1$–$_4$-Alkyl bezeichnet, wobei in dem Fall

in dem X 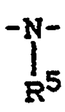 ist, die Gruppe 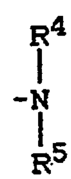 in

der Formel (I) die gleiche Bedeutung wie die Gruppe

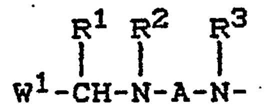

in der Formel (I) haben, kann,
Y N oder

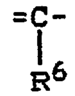

bezeichnen, worin $R^6$ Wasserstoff
$C_{1-4}$-Alkyl, $C_{6-10}$-Aryl, $C_{1-4}$-Alkylcarbonyl, Alkyoxycarbonyl mit $C_{1-4}$-Alkoxy oder Cyano bezeichnet,
Z Cyano oder Nitro bezeichnet,
$W^1$ eine 5– oder 6–gliedrige heterocyclische Gruppe mit 1 bis 3 Hetero-Atomen ausgewählt aus O, S und N, von denen wenigstens eines N ist, bezeichnet, wobei die Gruppe $W^1$ gegebenenfalls durch wenigstens eine Substituenten ausgewählt aus Halogen, $C_{1-4}$-Alkyl, $C_{1-4}$-Alkoxy, $C_{1-4}$-Alkylthio, $C_{1-4}$-Halogenalkyl und $C_{1-4}$-Halogenalkoxy substituierte ist, und
A Ethylen, das gegebenenfalls durch Methyl substituiert sein kann, oder Trimethylen, das gegebenenfalls durch Methyl substituiert sein kann, bezeichnet.

2. Verbindungen nach Anspruch 1, dadurch gekennzeichnet, daß
$R^1$, $R^2$ und $R^3$ Wasserstoff, Methyl oder Ethyl bezeichnen,
$R^4$ ein Wasserstoff-Atom, Methyl, Ethyl, Phenyl, Benzyl, Methoxy, Dimethylamino, 1-Ethoxyethyl, 1-Ethylthioethyl oder 2-Chloro–5–pyridylmethyl bezeichnet,
X S oder

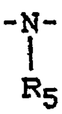

Wasserstoff oder Methyl bezeichnet,
N oder Y

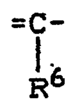

bezeichnen, worin $R^6$ Wasserstoff
Methyl, Phenyl, Acetyl, Ethoxycarbonyl oder Cyano bezeichnet,
Z Cyano oder Nitro bezeichnet,
$W^1$ eine 5- oder 6-gliedrige heterocyclische Gruppe mit 1 bis 2 Hetero-Atomen ausgewählt aus O, S und N, von denen wenigstens eines N ist, bezeichnet, wobei die Gruppe $W^1$ gegebenenfalls durch wenigstens einen Substituenten ausgewählt aus Fluoro, Chloro, Bromo, Methyl, Methoxy, Methylthio, Trifluoromethyl und Trifluoromethoxy substituiert ist, und
A Ethylen oder Trimethylen bezeichnet,

3. Alkylendiamin-Verbindung nach Ansprüchen 1 bis 2, ausgewählt aus den folgenden Verbindungen:
a) N-(2-Chloro-5-pyridylmethyl)-N-methyl-N′-(1-methylthio-2-nitrovinyl)ethylendiamin der folgenden Formel

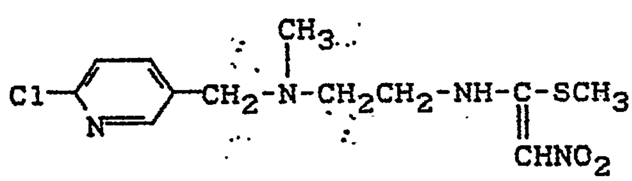

b) N-(2-Chloro-5-pyridylmethyl)-N-ethyl-N′-(1-methylthio-2-nitrovinyl)ethylendiamin der folgenden Formel

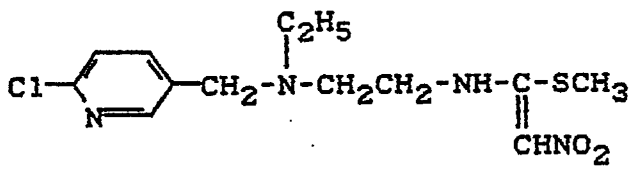

c) N-2-Chloro-5-pyridylmethyl)-N'-(1-mercapto-2-nitrovinyl)ethylendiamin der folgenden Formel

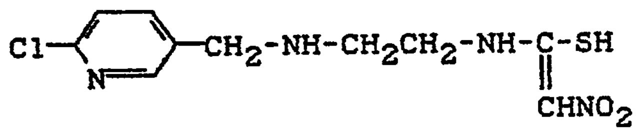

d) N-(2-Chloro-5-pyridylmethyl)-N'-(1-mercapto-2-nitrovinyl)trimethylendiamin der folgenden Formel

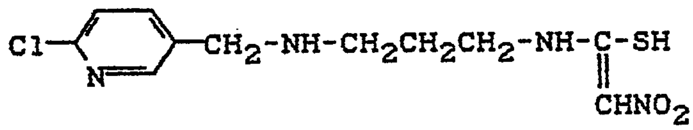

4. Verfahren zur Herstellung von Alkylendiaminen der Formel (I)

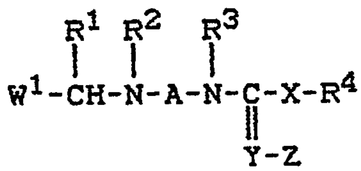 (I),

in welcher
$R^1$, $R^2$ und $R^3$ Wasserstoff oder $C_{1-4}$-Alkyl bezeichnen,
$R^4$ Wasserstoff, $C_{1-4}$-Alkyl, $C_{6-10}$-Aryl, Benzyl, Phenethyl, $C_{1-4}$-Alkoxy, Dialkylamino mit insgesamt 2 bis 6-Kohlenstoff-Atomen, Alkoxyalkyl mit insgesamt 2 bis 6 Kohlenstoff-Atomen, Alkylthioalkyl mit insgesamt 2 bis 4 Kohlenstoff-Atomen oder eine Gruppe der Formel $-CH_2-W^2$ bezeichnet, in der $W^2$ die gleichen Bedeutungen hat, wie sie hiernach für $W^1$ angegeben sind,
X S oder

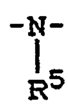

bezeichnet, worin $R^5$
Wasserstoff oder $C_{1-4}$-Alkyl bezeichnet, wobei in dem Fall,

in dem X 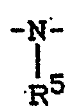 ist, die Gruppe 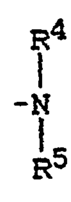 in

der Formel (I) die gleiche Bedeutung wie die Gruppe

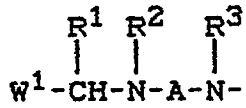

in der Formel (I) haben kann,
Y N oder

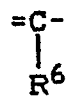

bezeichnet, worin $R^6$ Wasserstoff,
$C_{1-4}$-Alkyl, $C_{6-10}$-Aryl, $C_{1-4}$-Alkylcarbonyl, Alkyoxycarbonyl mit $C_{1-4}$-Alkoxy oder Cyano bezeichnet,
Z Cyano oder Nitro bezeichnet,
$W^1$ eine 5– oder 6–gliedrige heterocyclische Gruppe mit 1 bis 3 Hetero-Atomen ausgewählt aus O, S und N, von denen wenigstens eines N ist, bezeichnet, wobei die Gruppe $W^1$ gegebenenfalls durch wenigstens einen Substituenten ausgewählt aus Halogen, $C_{1-4}$-Alkyl, $C_{1-4}$-Alkoxy, $C_{1-4}$-Alkylthio, $C_{1-4}$-Halogenalkyl und $C_{1-4}$-Halogenalkoxy substituiert ist, und
A Ethylen, das gegebenenfalls durch Methyl substituiert sein kann, oder Trimethylen, das gegebenenfalls durch Methyl substituiert sein kann, bezeichnet, dadurch gekennzeichnet, daß

a) in dem Fall, in dem in der Formel (I)X S ist und $R^4$ andere Bedeutungen als "Alkoxy" und "Dialkylamino" in den Definitionen hat, wobei in diesem Fall in der nachstehenden Formel (III) $R^4$ durch das Symbol $R^7$ ersetzt ist, vorausgesetzt, daß die zwei Substituenten $R^7$ nicht gleichzeitig Wasserstoff sind, Verbindungen der Formel (II)

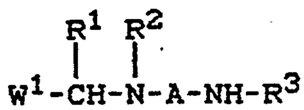 (II),

in der $R^1$, $R^2$, $R^3$, $W^1$ und A die angegebenen Bedeutungen haben, mit Verbindungen der Formel (III)

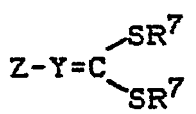 (III),

in der Y, Z und $R^7$ die angegebenen Bedeutungen haben, in Gegenwart inerter Lösungsmittel umgesetzt werden, oder

b) in dem Fall, in dem in der Formel (I)
X

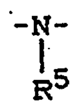

ist,
Verbindungen der Formel (IV)

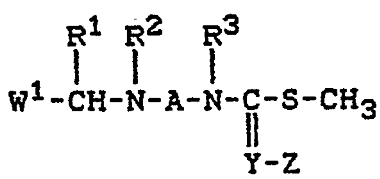 (IV),

in der $R^1$, $R^2$, $R^3$, Y, Z, $W^1$ und A die angegebenen Bedeutungen haben, mit Verbindungen der Formel (V)

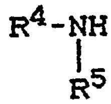 (V),

in der $R^4$ und $R^5$ die angegebenen Bedeutungen haben, in Gegenwart inerter Lösungsmittel umgesetzt werden, oder
c) in dem Fall, indem in der Formel (I)
X

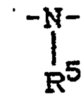

ist und Y N ist,
oben erwähnte Verbindungen der Formel (II) mit Verbindungen der Formel (IV)

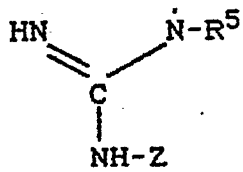 (VI)

in der $R^4$, $R^5$ und Z die angegebenen Bedeutungen haben, in Gegenwart inerter Lösungsmittel umgesetzt werden, oder
d) in dem Fall, in dem
Y

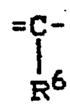

ist, X eine Einfachbindung
ist und $R^4$ durch das oben bezeichnete Symbol $R^7$ ersetzt ist, oben erwähnte Verbindungen der Formel (II) mit Verbindungen der Formel (VII)

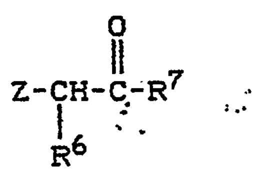 (VII)

in der $R^6$, $R^7$ und Z die angegebenen Bedeutungen haben, in Gegenwart inerter Lösungsmittel umgesetzt werden, oder
e) in dem Fall, in dem in der Formel (I) X S ist und $R^4$ andere Bedeutunge als "Wasserstoff", "Alkoxy" und "Dialkylamino" in den Definitionen hat, wobei in diesem Fall in den nachstehenden Formel (IX) $R^4$ durch das Symbol $R^8$ ersetzt ist,
Verbindungen der Formel (VIII)

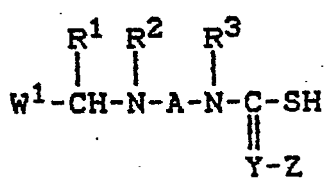 (VIII)

in der $R^1$, $R^2$, $R^3$, Y, Z und $W^1$ die angegenene Bedeutung haben, mit Verbindungen der Formel (IX)

Hal–$R^8$ (IX),

in der $R^8$ die angegebene Bedeutung hat und Hal ein Halogen bezeichnet, in Gegenwart inerter Lösungsmittel und gegebenenfalls in Gegenwart einer Base umgesetzt werden.

5. Insektizide Mittel, dadurch gekennzeichnet, daß sie wenigstens ein Alkylendiamin der Formel (I) enthalten.

6. Verfahren zur Bekämpfung schädlicher Insekten, dadurch gekennzeichnet, daß man Alkylendiamine der Formel (I) auf die schädlichen Insekten und/oder deren Lebensraum einwirken läßt.

7. Verwendung von Alkylendiaminen der Formel (I) zur Bekämpfung schädlicher Insekten.

8. Verfahren zur Herstellung insektizider Mittel, dadurch gekennzeichnet, daß Alkylendiamine der Formel (I) mit Streckmitteln und/oder grenzflächenaktiven Mitteln vermischt werden.

**Claims**

1. Alkylenediamines of the formula (1)

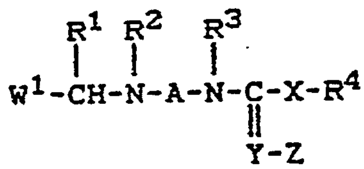

(I)

in which
$R^1$, $R^2$ and $R^3$ denote hydrogen $C_{1-4}$-alkyl,
$R^4$ denotes hydrogen, $C_{1-4}$-alkyl, $C_{6-10}$-aryl, benzyl, phenethyl, $C_{1-4}$-alkoxy, dialkylamino having altogether 2 to 6 carbon atoms, alkoxyalkyl having altogether 2 to 6 carbon atoms, alkylthioalkyl having altogether 2 to 4 carbon atoms or a group of the formula $-CH_2-W^2$, in which $W^2$ has the same meanings as are indicated hereafter for $W^1$-,
X denotes S or

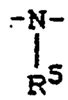

in which $R^5$ denotes hydrogen or
$C_{1-4}$-alkyl, where in the case in which X is

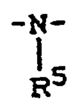

the group

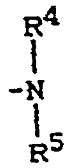

in the formula (I) can have the same meaning as the group

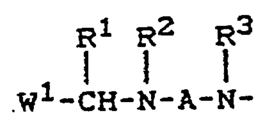

in the formula (I)
Y denotes N or

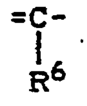

in which $R^6$ denotes hydrogen, $C_{1-4}$-alkyl, $C_{6-10}$-aryl, $C_{1-4}$-alkylcarbonyl, alkoxycarbonyl containing $C_{1-4}$-alkoxy, or cyano,
Z denotes cyano or nitro,
$W^1$ denotes a 5- or 6-membered heterocyclic group containing 1 to 3 heteroatoms selected from the group comprising O, S and N, of which at least one is N, where the group $W^1$ is optionally substituted by at least one substituent selected from the group comprising halogen, $C_{1-4}$ alkyl, $C_{1-4}$-alkoxy, $C_{1-4}$-alkylthio, $C_{1-4}$-halogenoalkyl and $C_{1-4}$-halogenoalkoxy, and
A denotes ethylene which can optionally be substituted by methyl, or trimethylene which can optionally be substituted by methyl.

2. Compounds according to Claim 1, characterized in that
$R^1$, $R^2$ and $R^3$ denote hydrogen, methyl or ethyl,
$R^4$ denotes a hydrogen atom, methyl, ethyl, phenyl, benzyl, methoxy, dimethylamino, 1-ethoxyethyl, 1-ethylthioethyl or 2-chloro-5-pyridylmethyl,
X denotes S or

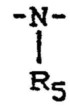

in which $R^5$ denotes hydrogen or methyl,
Y denotes N or

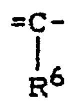

in which $R^6$ denotes hydrogen, methyl, phenyl, acetyl, ethoxycarbonyl or cyano,
Z denotes cyano or nitro,
$W^1$ denotes a 5- or 6-membered heterocyclic group having 1 or 2 heteroatoms selected from the group comprising O, S and N, of which at least one is N, where the group $W^1$ is optionally substituted by at least one substituent selected from the group comprising fluoro, chloro, bromo, methyl, methoxy, methylthio, trifluoromethyl and trifluoromethoxy, and
A denotes ethylene or trimethylene.

3. Alkylenediamine compounds according to Claim 1 or 2, selected from the following compounds:

a) N-(2-chloro-5-pyridylmethyl)-N-methyl-N'-(1-methylthio-2-nitrovinyl)ethylenediamine of the following formula

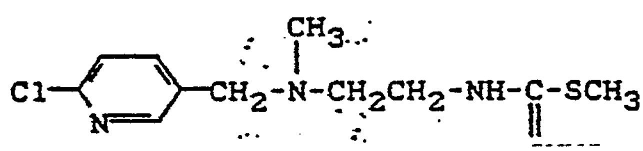

b) N-(2-chloro-5-pyridylmethyl)-N-ethyl-N'-(1-methylthio-2-nitrovinyl)ethylenediamine of the following formula ,

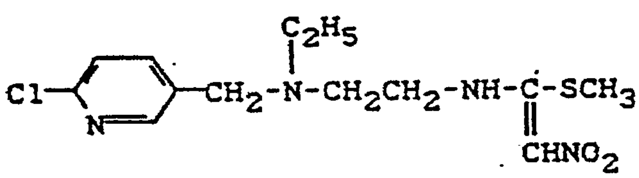

c) N-2-chloro-5-pyridylmethyl)-N'-(1-mercapto-2-nitrovinyl)ethylenediamine of the following formula

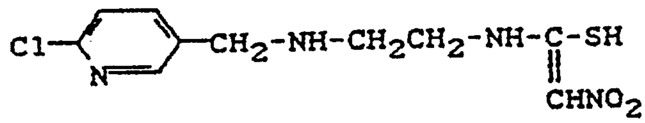

d) N-(2-chloro-5-pyridylmethyl)-N'-(1-mercapto-2-nitrovinyl)trimethylenediamine of the following formula

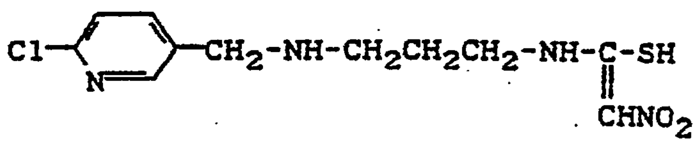

4. Process for the preparation of alkylenediamins of the formula (I)

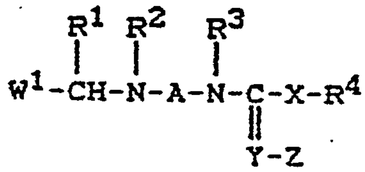

(I)

in which
$R^1$, $R^2$ and $R^3$ denote hydrogen or $C_{1-4}$-alkyl
$R^4$ denotes hydrogen, $C_{1-4}$-alkyl, $C_{6-10}$-aryl, benzyl, phenethyl, $C_{1-4}$-alkoxy, dialkylamino having altogether 2 to 6 carbon atoms, alkoxyalkyl having altogether 2 to 6 carbon atoms, alkylthioalkyl having altogether 2 to 4 carbon atoms or a group of the formula $-CH_2-W^2$, in which $W^2$ has the same meanings as are indicated hereafter for $W^1$,
X denotes S or

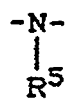

in which $R^5$ denotes hydrogen or $C_{1-4}$-alkyl, where in the case in which X is

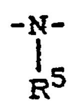

the group

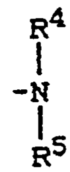

in the formula (I) can have the same meaning as the group

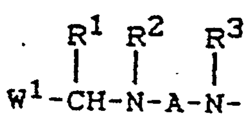

in the formula (I),

Y denotes N or

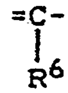

in which R6 denotes hydrogen, $C_{1-4}$-alkyl, $C_{6-10}$-aryl, $C_{1-4}$-alkylcarbonyl, alkoxycarbonyl containing $C_{1-4}$-alkoxy, or cyano,
Z denotes cyano or nitro,
$W^1$ denotes a 5- or 6-membered heterocyclic group containing 1 to 3 heteroatoms selected from the group comprising O, S and N, of which at least one is N, where the group $W^1$ is optionally substituted by at least one substituent selected from the group comprising halogen, $C_{1-4}$-alkyl, $C_{1-4}$-alkoxy, $C_{1-4}$-alkylthio, $C_{1-4}$-halogenoalkyl and $C_{1-4}$-halogenoalkoxy, and
A denotes ethylene which can optionally be substituted by methyl, or trimethylene which can optionally be substituted by methyl, characterized in that

a) in the case in which X is S in the formula (1) and $R^4$ has meanings other than "alkoxy " and "dialkylamino" in the definitions, where in this case in the formula (III) below, $R^4$ is replaced by the symbol $R^7$ provided that the two substituents $R^7$ are not simultaneously hydrogen, compounds of the formula (II)

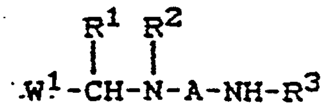 (II),

in which $R^1$, $R^2$, $R^3$, $W^1$ and A have the meanings indicated, are reacted with compounds of the formula (III)

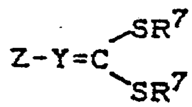 (III)

in which Y, Z and $R^7$ have the meanings indicated, in the presence of inert solvents, or
b) in the case in which in the formula (I) X is

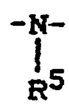

compounds of the formula (IV)

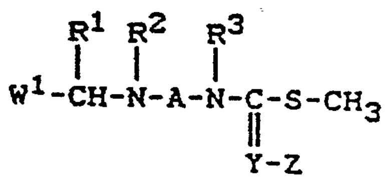 (IV)

in which $R^1$, $R^2$, $R^3$, Y, Z, $W^1$ and A have the meanings indicated, are reacted with compounds of the formula (V)

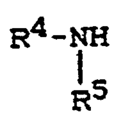 (V)

in which $R^4$ and $R^5$ have the meanings indicated, in the presence of inert solvents, or
c) in the case which in the formula (I)

X is

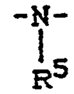

and Y is N,
the abovementioned compounds of the formula (II) are reacted with compounds of the formula (VI)

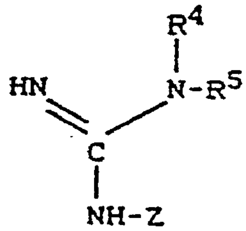 (VI),

in which $R^4$, $R^5$ and Z have the meanings indicated, in the presence of inert solvents, or
d) in the case in which Y is

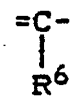 .

X is a single bond and $R^4$ is replaced by the symbol $R^7$ indicated above – abovementioned compounds of the formula (II) are reacted with compounds of the formula (VII)

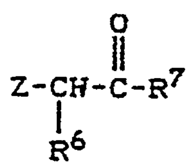

in which $R^6$, $R^7$ and Z have the meanings indicated, in the presence of inert solvents, or
e) in the case in which in the formula (I) X is S and $R^4$ has meanings other than "hydrogen", "alkoxy" and "dialkylamino" in the definitions, where in this case in the formula (IX) below, $R^4$ is replaced by the symbol $R^8$,
compounds of the formula (VIII)

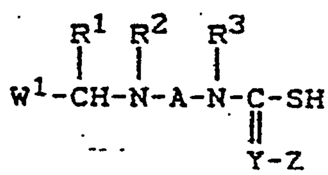 (VIII)

in which $R^1$, $R^2$, $R^3$, Y, Z and $W^1$ have the meanings indicated, are reacted with compounds of the formula (IX)

Hal-$R^8$ (IX)

in which $R^8$ has the meaning indicated and Hal denotes a halogen, in the presence of inert solvents and, if appropriate, in the presence of a base.
5. Insecticidal agents, characterized in that they contain at least one alkylenediamine of the formula (I).
6. Process for combating harmful insects characterized in that alkylenediamines of the formula (I) are allowed to act on the harmful insects and/or their environment.
7. Use of alkylenediamines of the formula (I) for combating harmful insects.
8. Method for the production of insecticidal agents, characterized in that alkylenediamines of the formula (I) are mixed with extenders and/or surface-active agents.

**Revendications**

1. Alkylènediamines de formule (I)

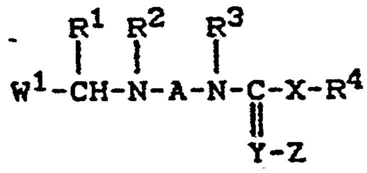

(I)

dans laquelle
$R^1$, $R^2$ et $R^3$ représentent l'hydrogène ou des groupes alkyle en $C_1$ à $C_4$,
$R^4$ est l'hydrogène, un groupe alkyle en $C_1$ à $C_4$, aryle en $C_6$ à $C_{10}$, benzyle, phénéthyle, alkoxy en $C_1$ à $C_4$, dialkylamino ayant au total 2 à 6 atomes de carbone, alkoxyalkyle ayant au total 2 à 6 atomes de carbone, alkylthioalkyle ayant au total 2 à 4 atomes de carbone ou un groupe de formule $-CH_2-W^2$ dans laquelle $W^2$ a les mêmes définitions que celles qui sont indiquées ci-après pour $W^1$,
X représente S ou un groupe

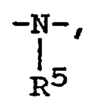

dans lequel $R^5$ est l'hydrogène ou un groupe alkyle en $C_1$ à $C_4$, et au cas où X représente

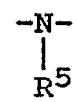

le groupe

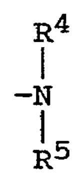

dans la formule (I) peut avoir la même définition que le groupe

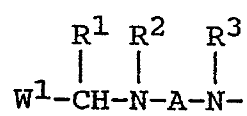

dans la formule (I),
Y représente N ou un groupe

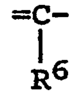

dans lequel $R^6$ est l'hydrogène, un groupe alkyle en $C_1$ à $C_4$, aryle en $C_6$ à $C_{10}$, (alkyle en $C_1$ à $C_4$)-carbonyle, alkoxycarbonyle ayant un radical alkoxy en $C_1$ à $C_4$, ou cyano,
Z est un groupe cyano ou nitro, $W^1$ est un groupe hétérocyclique pentagonal ou hexagonal ayant 1 à 3 hétéro-atomes choisis entre des atomes de O, S et N, dont l'un au moins est un atome de N, le groupe $W^1$ étant éventuellement substitué par au moins un substituant choisi entre un halogène, un groupe alkyle en $C_1$ à $C_4$, un groupe alkoxy en $C_1$ à $C_4$, un groupe alkylthio en $C_1$ à $C_4$, un groupe halogénalkyle en $C_1$ à $C_4$ et un groupe halogénalkoxy en $C_1$ à $C_4$, et
A est un groupe éthylène qui peut être substitué, le cas échéant, par un radical méthyle, ou un groupe triméthylène qui peut être substitué, le cas échéant, par un radical méthyle.

2. Composés suivant la revendication 1, caractérisés en ce que $R^1$, $R^2$ et $R^3$ désignent l'hydrogène, des radicaux méthyle ou éthyle, $R^4$ est un atome d'hydrogène, un groupe méthyle, éthyle, phényle, benzyle, méthoxy, diméthylamino, 1-éthoxyéthyle, 1-éthylthioéthyle ou 2-chloro-5-pyridylméthyle, X représente S ou un groupe

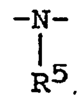

dans lequel $R^5$ est l'hydrogène ou un groupe méthyle, Y représente N ou un groupe

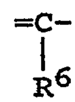

dans lequel $R^6$ est l'hydrogène, un groupe méthyle, phényle, acétyle, éthoxycarbonyle ou cyano, Z est un groupe cyano ou nitro, $W^1$ est un groupe hétérocyclique pentagonal ou hexagonal ayant 1 ou 2 hétéro-atomes choisis entre les atomes d'oxygène, de soufre et d'azote dont l'un au moins est un atome d'azote, le groupe $W^1$ étant substitué, le cas échéant, par au moins un substituant choisi entre les radicaux fluoro, chloro, bromo, méthyle, méthoxy, méthylthio, trifluorométhyle et trifluorométhoxy, et A est un groupe éthylène ou triméthylène.

3. Alkylènediamines suivant les revendications 1 et 2, choisies parmi les composés suivants:

a) N-(2-chloro-5-pyridylméthyl)-N-méthyl-N'-(1-méthylthio-2-nitrovinyl)éthylènediamine de formule suivante

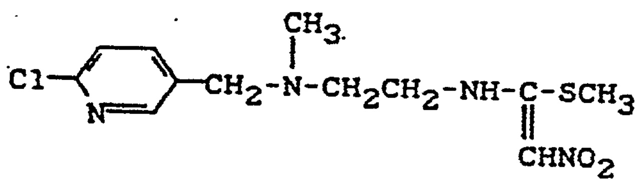

b) N-(2-chloro-5-pyridylméthyl)-N-éthyl-N'-(1-méthylthio-2-nitrovinyl)éthylènediamine de formule suivante

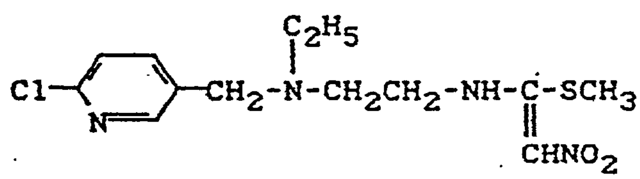

c) N-(2-chloro-5-pyridylméthyl)-N'-(1-mercapto-2-nitrovinyl)éthylènediamine de formule suivante

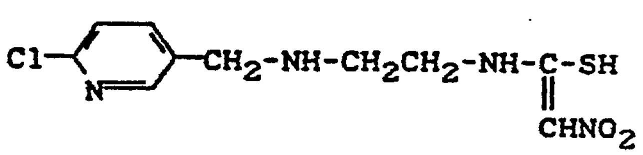

d) N-(2-chloro-5-pyridylméthyl)-N'-(1-mercapto-2-nitrovinyl)triméthylènediamine de formule suivante

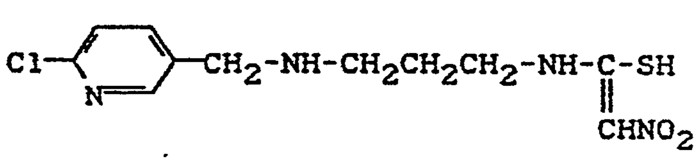 .

4. Procédé de préparation d'alkylènediamines de formule (I)

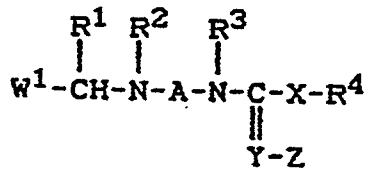 (I)

dans laquelle
$R^1$, $R^2$ et $R^3$ représentent l'hydrogène ou des groupes alkyle en $C_1$ à $C_4$,
$R^4$ est l'hydrogène, un groupe alkyle en $C_1$ à $C_4$, aryle en $C_6$ à $C_{10}$, benzyle, phénéthyle, alkoxy en $C_1$ à $C_4$, dialkylamino ayant au total 2 à 6 atomes de carbone, alkoxyalkyle ayant au total 2 à 6 atomes de carbone, alkylthioalkyle ayant au total 2 à 4 atomes de carbone ou un groupe de formule $-CH_2-W^2$ dans laquelle $W^2$ a les mêmes définitions que celles qui sont indiquées ci-après pour $W^1$,
X représente S ou un groupe

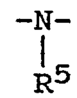

dans lequel $R^5$ est l'hydrogène ou un groupe alkyle en $C_1$ à $C_4$, et au cas où X représente

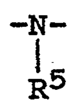

le groupe

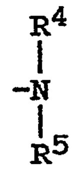

dans ls formule (I) peut avoir la même définition que le groupe

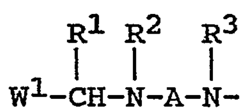

dans la formule (I),
Y représente N ou un groupe

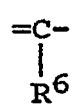

dans lequel $R^6$ est l'hydrogène, un groupe alkyle en $C_1$ à $C_4$, aryle en $C_6$ à $C_{10}$, (alkyle en $C_1$ à $C_4$)-carbonyle, alkoxycarbonyle ayant un radical alkoxy en $C_1$ à $C_4$, ou cyano,
Z est un groupe cyano ou nitro,
$W^1$ est un groupe hétérocyclique pentagonal ou hexagonal ayant 1 à 3 hétéro-atomes choisis entre des atomes de O, S et N, dont l'un au moins est un atome de N, le groupe $W^1$ étant éventuellement substitué par au moins un substituant choisi entre un halogène, un groupe alkyle en $C_1$ à $C_4$, un groupe alkoxy en $C_1$ à $C_4$, un groupe alkylthio en $C_1$ à $C_4$, un groupe halogénalkyle en $C_1$ à $C_4$ et un groupe halogénalkoxy en $C_1$ à $C_4$, et
A est un groupe éthylène qui peut être substitué, le cas échéant, par un radical méthyle, ou un groupe triméthylène qui peut être substitué, le cas échéant, par un radical méthyle, caractérisé en ce que

a) au cas où dans la formule (I), X représente S et $R^4$ a des valeurs autres que "alkoxy" et "dialkylamino" indiquées dans les définitions, $R^4$ étant alors remplacé par le symbole $R^7$ dans la formule (III) ci-après, sous réserve que les deux substituants $R^7$ ne représentent pas simultanément de l'hydrogène, on fait réagir des composés de formule (II)

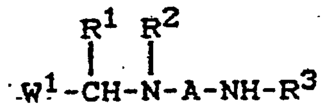 (II),

dans laquelle $R^1$, $R^2$, $R^3$, $W^1$ et A ont les définitions indiquées, avec des composés de formule (III)

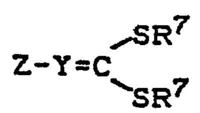 (III),

dans laquelle Y, Z et $R^7$ ont les définitions indiquées, en présence de solvant inertes, ou bien

b) au cas où dans la formule (I), X représente

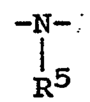

on fait réagir des composés de formule (IV)

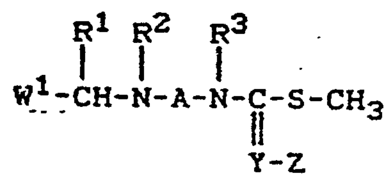 (IV)

dans laquelle $R^1$, $R^2$, $R^3$, Y, Z, $W^1$ et A ont les définitions indiquées, avec des composés de formule (V)

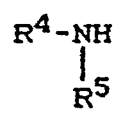 (V)

dans laquelle $R^4$ et $R^5$ ont les définitions indiquées, en présence de solvants inertes, ou bien

c) au cas où dans la formule (I), X représente

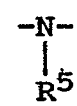

et Y représente N, on fait réagir des composés mentionnés ci-dessus de formule (II) avec des composés de formule (IV))

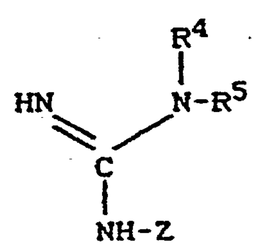 (VI)

dans laquelle $R^4$, $R^5$ et Z ont les définitions indiquées, en présence de solvants inertes, ou bien

d) au cas où Y représente

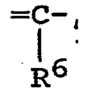

X est une liaison simple et $R^4$ est remplacé par le symbole $R^7$ indiqué ci-dessus, on fait réagir des composés mentionnés ci-dessus de formule (II) avec des composés de formule (VII)

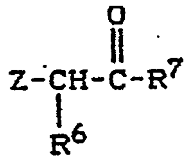

(VII)

dans laquelle $R^6$, $R^7$ et Z ont les définitions indiquées, en présence de solvants inertes, ou bien

e) au cas où dans la formule (I), X représente S et $R^4$ a des valeurs autres que "hydrogène", "alkoxy" et "dialkylamino" dans les définitions indiquées, auquel cas dans la formule (IX) $R^4$est remplacé par le symbole $R^8$, on fait réagir des composés de formule (VIII)

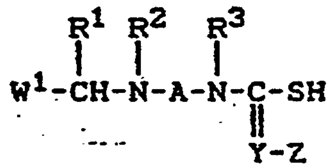

(VIII).

dans laquelle $R^1$, $R^2$, $R^3$, Y, Z et $W^1$ ont les définitions indiquées, avec des composés de formule (IX)
Hal–$R^8$ (IX)⁻,
dans laquelle $R^8$ a la définition indiquée et Hal désigne un halogène, en présence de solvants inertes et, le cas échéant, en présence d'une base.

5. Compositions insecticides, caractérisées en ce qu'elles contiennent au moins une alkylènediamine de formule (I).

6. Procédé pour combattre des insectes parasites, caractérisé en ce qu'on fait agir des alkylènediamines de formule (I) sur les insectes parasites et/ou sur leur milieu.

7. Utilisation d'alkylènediamines de formule (I) pour combattre des insectes nuisibles.

8. Procédé de préparation de compositions insecticides, caractérisé en ce qu'on mélange des alkylènediamines de formule (I) avec des diluants et/ou des agents tensio-actifs.